# EUROPEAN PATENT APPLICATION

(11) **EP 4 538 277 A1**
(43) Date of publication of application: **16.04.2025**
(21) Application number: 23819042.5
(22) Date of filing: 05.06.2023
(51) Int. Cl.: C07D 493/00, A61K 31/352, A61P 11/06, A61P 11/08, A61P 19/02, A61P 3/10, A61P 37/00, A61P 13/12

(54) **PHARMACEUTICALLY ACCEPTABLE SALT OF BENZO[C]CHROMAN COMPOUND AND POLYMORPHIC FORM AND USE OF PHARMACEUTICALLY ACCEPTABLE SALT**

(30) Priority: 07.06.2022 CN 202210640471
(71) Applicant: Reistone Biopharma Company Limited, Shanghai 201210 (CN)
(72) Inventor: SUN, Longwei, Shanghai 201210 (CN); HOU, Yanting, Shanghai 201210 (CN); ZHU, Jiajun, Shanghai 201210 (CN); YANG, Guang, Shanghai 201210 (CN); HAO, Xin, Shanghai 201210 (CN); LUO, Zhiyang, Shanghai 201210 (CN)
(74) Representative: Potter Clarkson
(86) International application number: PCT/CN2023/098204
(87) International publication number: WO 2023/236877

(57) **Abstract**

A pharmaceutically acceptable salt of a benzo[c]chroman compound and a polymorphic form and use of the pharmaceutically acceptable salt. The pharmaceutically acceptable salt is selected from hydrochloride, methanesulfonate, phosphate, L-tartrate, maleate, p-toluenesulfonate, sulfate, fumarate, succinate, citrate, malate, and hydrobromide. The pharmaceutically acceptable salt and the polymorphic form thereof improve the bioavailability and the stability, and as a cathepsin C inhibitor, can be used for treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, sinusitis, hidradenitis suppurativa, or cancer.

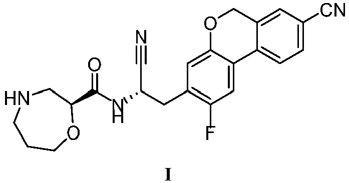

## Description

### FIELD OF THE INVENTION

The present disclosure belongs to the field of pharmaceutical technology, and relates to pharmaceutically acceptable salts of benzo[c]chromane compounds and polymorphs and uses thereof.

### BACKGROUND OF THE INVENTION

Cathepsins are a class of proteolytic enzymes widely present in lysosomes of various tissue cells. According to their structures and catalytic types, cathepsins are divided into three classes: serine proteases (cathepsins A and G), aspartic proteases (cathepsins D and E), and cysteine proteases. Among them, cysteine proteases are the largest family of cathepsins and include 11 proteases: cathepsins B, C, F, H, K, L, O, S, W, V, and Z.

Cathepsin C is also known as dipeptidyl peptidase I or "DPP1". DPP1 is constitutively expressed in many tissues with the highest levels in the lung, kidney, liver, and spleen. Several recently published studies have described the role played by cathepsin C in certain inflammatory processes. For example, Adkison et al., J Clin Invest. 2002 Feb; 109(3):363-71; Tinh et al., Archives of Biochemistry and Biophysics. 2002 403:160-170, it can be seen from these studies that cathepsin C is co-expressed in granules with certain serine proteases, and functions to process the precursor forms of these proteases into active forms, which are then released from inflammatory cell granules recruited to sites of inflammation. Once activated, these proteases have numerous functions, including the degradation of various extracellular matrix components, which together can propagate tissue damage and chronic inflammation.

WO 2004/110988 relates to certain nitrile derivatives and the use thereof as DPP1 inhibitors.

WO 2009/074829 relates to peptidyl nitriles and the use thereof as DPP1 inhibitors.

WO 2010/128324 relates to α-aminoamide nitriles and the use thereof as DPP1 inhibitors.

WO 2012/119941 relates to peptidyl nitrile compounds and the use thereof as DPP1 inhibitors.

WO 2013/041497 relates to N-[1-cyano-2-(phenyl)ethyl]-2-azabicyclo[2.2.1]heptane-3-carboxamide and the use thereof as a DPP1 inhibitor.

WO 2001/096285 and WO 2003/048123 relate to β-aminoamide nitriles has inhibitory activity against cysteine proteases.

WO 2015/110826 relates to α-aminoamide nitriles and the use thereof as DPP1 inhibitors.

WO 2022/117059 provides a cathepsin C inhibitor, chemically named (*S*)-N-((*S*)-1-cyano-2-(8-cyano-2-fluoro-6*H*-benzo[*c*]chromen-3-yl)ethyl)-1,4-oxazepane-2-carboxamide, with the structure shown in formula I.

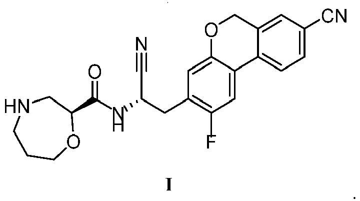

The crystal form of a pharmaceutically active ingredient often affects the chemical stability of a drug, and different crystallization and storage conditions may lead to changes in the crystal structure of a compound, sometimes accompanied by production of other crystal forms. In general, an amorphous drug product does not have a regular crystal structure, and often has other defects such as poor product stability, fine precipitation, difficult filtration, easy agglomeration, poor flowability and the like. The polymorphism of a drug has different requirements on product storage, production, and scaleup. Therefore, it is necessary to conduct in-depth research on crystal forms of the aforementioned compound so as to improve various properties of the aforementioned compound.

### SUMMARY OF THE INVENTION

The present invention provides a pharmaceutically acceptable salt of a compound of formula I, wherein the pharmaceutically acceptable salt is an acid addition salt selected from the group consisting of hydrochloride salt, methanesulfonate salt, phosphate salt, L-tartrate salt, maleate salt, p-toluenesulfonate salt, sulfate salt, fumarate salt, succinate salt, citrate salt, malate salt and hydrobromide salt, and is preferably in the form of a crystal.

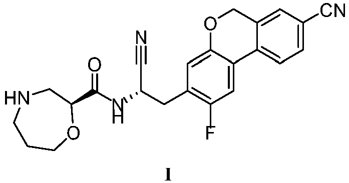

In some embodiments, provided is the pharmaceutically acceptable salt of the compound of formula I, wherein the stoichiometric ratio of the compound of formula I to the acid molecule or acid group from 1:0.5 to 1:3, preferably 1:0.5, 1:1, 1:1.2, 1:2 or 1:3, more preferably 1:1.

In some embodiments, provided is the pharmaceutically acceptable salt of the compound of formula I, wherein the pharmaceutically acceptable salt of the compound of formula I is hydrochloride salt, and the stoichiometric ratio of the compound of formula I to the hydrochloric acid group is 1: 1.

The present disclosure also provides a method for preparing the pharmaceutically acceptable salt above, comprising a step of salifying the compound of formula I with an acid. In some embodiments, the step of salifying the compound of formula I with an acid is carried out in a solvent selected from one or more of dichloromethane, N,N-dimethylformamide, acetonitrile, methanol, ethanol, isopropanol, tetrahydrofuran, acetone, 1,4-dioxane, water, dimethyl sulfoxide, and ethyl acetate, preferably acetone, methanol, ethanol, ethanol/water, isopropanol/water, ethyl acetate, acetonitrile, tetrahydrofuran, dimethyl sulfoxide/water, 1,4-dioxane, methanol/water, N,N-dimethylformamide, and dichloromethane.

The present disclosure also provides crystal form A of hydrochloride salt of the compound of formula I, wherein the X-ray powder diffraction pattern thereof represented by diffraction angle 2*θ* angle has characteristic peaks at 7.043, 12.430, 14.356, 14.840 and 15.250, wherein the error range of the 2*θ* angle is ±0.20.

In some embodiments, provided is the crystal form A of hydrochloride salt of the compound of formula I, wherein the X-ray powder diffraction pattern thereof represented by diffraction angle 2*θ* angle has characteristic peaks at 7.043, 8.719, 10.737, 12.430, 14.356, 14.840, 15.250 and 17.686, wherein the error range of the 2*θ* angle is ±0.20.

In some embodiments, provided is the crystal form A of hydrochloride salt of the compound of formula I, wherein the X-ray powder diffraction pattern thereof represented by diffraction angle 2*θ* angle has characteristic peaks at 7.043, 8.719, 10.737, 12.430, 14.356, 14.840, 15.250, 17.686, 21.697, 22.305, 25.635 and 27.433, wherein the error range of the 2*θ* angle is ±0.20.

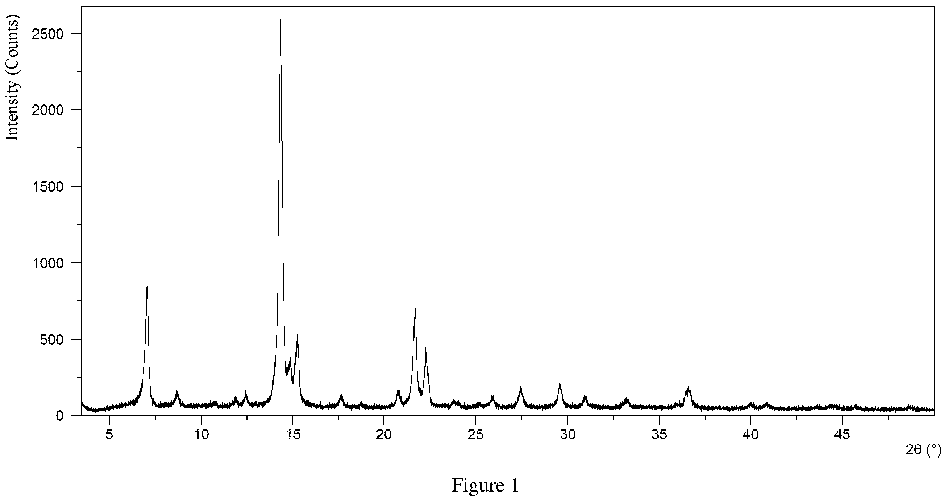

In some embodiments, provided is the crystal form A of hydrochloride salt of the compound of formula I, wherein the X-ray powder diffraction pattern thereof represented by diffraction angle 2θ angle is as shown in Figure 1.

In some embodiments, provided is the crystal form A of hydrochloride salt of the compound of formula I, wherein the differential scanning calorimetry (DSC) spectrum thereof has an endothermic peak at 268°C, with the error range of ±2°C.

The present disclosure also provides a method for preparing crystal form A of hydrochloride salt of the compound of formula I above, comprising the steps of:
a) dissolving hydrochloride salt of the compound of formula I in Solvent A;
b) performing stirring.

In some embodiments, the preparation method further comprises the step(s) of performing centrifuging, washing and/or drying.

In some embodiments, in the method for preparing crystal form A of hydrochloride salt of the compound of I, the Solvent A is selected from the group consisting of water, C₁₋₄ ketone solvent, C₁₋₄ alcohol solvent, mixed solvent of water and C₁₋₄ alcohol solvent, C₁₋₄ ester solvent, C₁₋₄ nitrile solvent, tetrahydrofuran, mixed solvent of dimethyl sulfoxide and water, and 1,4-dioxane, preferably selected from the group consisting of acetone, methanol, ethanol, ethanol/water, isopropanol/water, ethyl acetate, acetonitrile, tetrahydrofuran, dimethyl sulfoxide/water, 1,4-dioxane, and methanol/water.

In some embodiments, in the method for preparing crystal form A of hydrochloride salt of the compound of formula I, each milligram of the compound of formula I is dissolved infrom 0.01 to 0.05 ml of the Solvent A, more preferably each milligram of the compound of formula I is dissolved in from 0.01 to 0.03 ml of the Solvent A.

In some embodiments, in the method for preparing crystal form A of hydrochloride salt of the compound of formula I, the mixed solvent of water and alcohol solvent is a mixed solvent of water and methanol, wherein the molar ratio of water to methanol is from 0.10 to 0.95, preferably 0.14, 0.26, 0.37, 0.47, 0.57, 0.66, 0.74, 0.82 and 0.90.

The present disclosure also provides crystal form A' of hydrochloride salt of the compound of formula I above, wherein the X-ray powder diffraction pattern thereof represented by diffraction angle 2θ angle has characteristic peaks at 8.452, 12.476, 15.884, 17.037, 17.227, 22.328 and 23.566, wherein the error range of the 2θ angle is ±0.20.

In some embodiments, provided is the crystal form A' of hydrochloride salt of the compound of formula I, wherein the X-ray powder diffraction pattern thereof represented by diffraction angle 2*θ* angle has characteristic peaks at 8.452, 10.537, 12.476, 15.884, 17.037, 17.227, 20.784, 22.328, 23.566 and 27.567, wherein the error range of the 2*θ* angle is ±0.20.

In some embodiments, provided is the crystal form A' of hydrochloride salt of the compound of formula I, wherein the X-ray powder diffraction pattern thereof represented by diffraction angle 2*θ* angle has characteristic peaks at 8.452, 10.537, 12.476, 13.296, 14.332, 15.884, 17.037, 17.227, 20.784, 22.328, 23.566 and 27.567, wherein the error range of the 2*θ* angle is ±0.20.

In some embodiments, provided is the crystal form A' of hydrochloride salt of the compound of formula I, wherein the X-ray powder diffraction pattern thereof represented by diffraction angle 2*θ* angle has characteristic peaks at 8.452, 10.537, 12.476, 13.296, 14.332, 15.884, 17.037, 17.227, 20.784, 21.329, 22.328, 23.566, 24.497, 25.221 and 27.567, wherein the error range of the 2*θ* angle is ±0.20.

In some embodiments, provided is the crystal form A' of hydrochloride salt of the compound of formula I, wherein the DSC spectrum thereof has an endothermic peak at 161°C, with the error range of ±2°C.

The present disclosure also provides crystal form B of methanesulfonate salt of the compound of formula I above, wherein the X-ray powder diffraction pattern thereof represented by diffraction angle 2*θ* angle has characteristic peaks at 6.717, 8.783, 13.969, 15.902, 16.647 and 17.515, wherein the error range of the 2*θ* angle is ±0.20.

In some embodiments, provided is the crystal form B of methanesulfonate salt of the compound of formula I, wherein the X-ray powder diffraction pattern thereof represented by diffraction angle 2*θ* angle has characteristic peaks at 6.717, 8.105, 8.783, 13.969, 15.902, 16.647 and 17.515, wherein the error range of the 2*θ* angle is ±0.20.

In some embodiments, provided is the crystal form B of methanesulfonate salt of the compound of formula I, wherein the X-ray powder diffraction pattern thereof represented by diffraction angle 2*θ* angle has characteristic peaks at 6.717, 8.105, 8.783, 13.969, 15.902, 16.647, 17.515, 20.446, 21.069, 21.645 and 24.665, wherein the error range of the 2*θ* angle is ±0.20.

In some embodiments, provided is the crystal form B of methanesulfonate salt of the compound of formula I, wherein the X-ray powder diffraction pattern thereof represented by diffraction angle 2*θ* angle has characteristic peaks at 6.717, 8.105, 8.783, 13.969, 15.902, 16.647, 17.515, 20.446, 21.069, 21.645, 23.549, 24.665, 26.284, 27.289 and 27.667, wherein the error range of the 2*θ* angle is ±0.20.

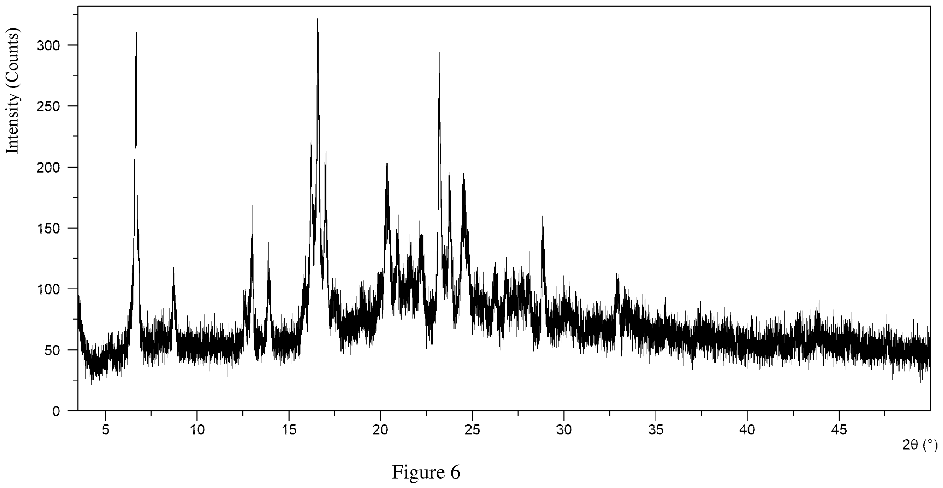

In some embodiments, provided is the crystal form B of methanesulfonate salt of the compound of formula I, wherein the X-ray powder diffraction pattern thereof represented by diffraction angle 2*θ* angle is as shown in Figure 6.

In some embodiments, provided is the crystal form B of methanesulfonate salt of the compound of formula I, wherein the DSC spectrum thereof has an endothermic peak at 161°C, with the error range of ±2°C.

The present disclosure also provides crystal form C of methanesulfonate salt of the compound of formula I above, wherein the X-ray powder diffraction pattern thereof represented by diffraction angle 2*θ* angle has characteristic peaks at 7.747, 11.163, 12.676, 15.268, 16.824, 18.549 and 19.759, wherein the error range of the 2*θ* angle is ±0.20.

In some embodiments, provided is the crystal form C of methanesulfonate salt of the compound of formula I, wherein the X-ray powder diffraction pattern thereof represented by diffraction angle 2*θ* angle has characteristic peaks at 4.522, 7.747, 11.163, 12.676, 15.268, 16.824, 18.549 and 19.759, wherein the error range of the 2*θ* angle is ±0.20.

In some embodiments, provided is the crystal form C of methanesulfonate salt of the compound of formula I, wherein the X-ray powder diffraction pattern thereof represented by diffraction angle 2*θ* angle has characteristic peaks at 4.522, 7.747, 11.163, 12.676, 15.268, 16.824, 18.549, 19.759, 21.460, 24.637 and 25.497, wherein the error range of the 2*θ* angle is ±0.20.

In some embodiments, provided is the crystal form C of methanesulfonate salt of the compound of formula I, wherein the X-ray powder diffraction pattern thereof represented by diffraction angle 2*θ* angle has characteristic peaks at 4.522, 7.747, 11.163, 12.676, 15.268, 16.824, 18.549, 19.759, 21.460, 22.539, 24.637 and 25.497, wherein the error range of the 2*θ* angle is ±0.20.

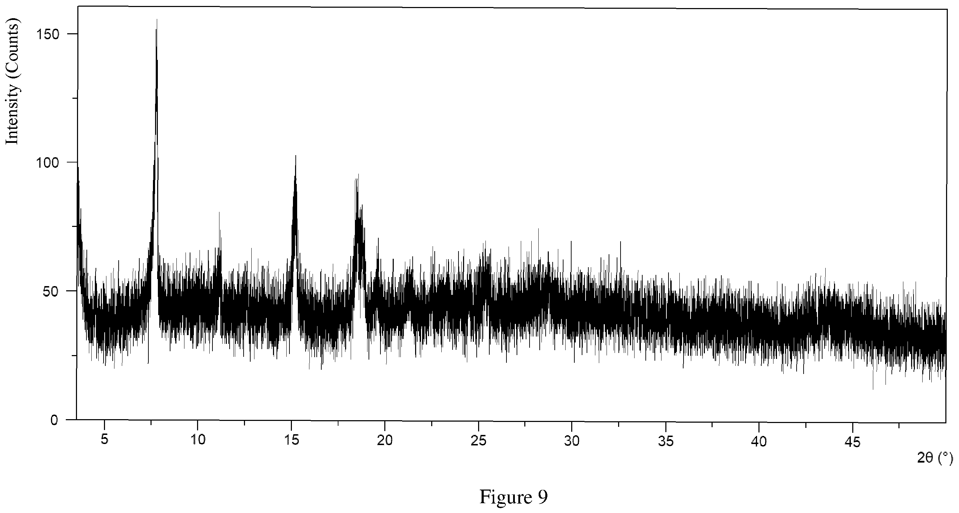

In some embodiments, provided is the crystal form C of methanesulfonate salt of the compound of formula I, wherein the X-ray powder diffraction pattern thereof represented by diffraction angle 2*θ* angle is as shown in Figure 9.

In some embodiments, provided is the crystal form C of methanesulfonate salt of the compound of formula I, wherein the DSC spectrum thereof has an endothermic peak at 194°C, with the error range of ±2°C.

The present disclosure also provides crystal form D of phosphate salt of the compound of formula I above, wherein the X-ray powder diffraction pattern thereof represented by diffraction angle 2*θ* angle has characteristic peaks at 9.593, 12.831, 13.464, 15.666, 18.161 and 19.245, wherein the error range of the 2*θ* angle is ±0.20.

In some embodiments, provided is the crystal form D of phosphate salt of the compound of formula I, wherein the X-ray powder diffraction pattern thereof represented by diffraction angle 2*θ* angle has characteristic peaks at 8.491, 9.593, 12.831, 13.464, 13.954, 14.943, 15.666, 16.616, 17.259, 18.161 and 19.245, wherein the error range of the 2*θ* angle is ±0.20.

In some embodiments, provided is the crystal form D of phosphate salt of the compound of formula I, wherein the X-ray powder diffraction pattern thereof represented by diffraction angle 2*θ* angle has characteristic peaks at 4.213, 6.650, 8.491, 9.593, 10.897, 12.831, 13.464, 13.954, 14.943, 15.666, 16.616, 17.259, 18.161 and 19.245, wherein the error range of the 2*θ* angle is ±0.20.

In some embodiments, provided is the crystal form D of phosphate salt of the compound of formula I, wherein the X-ray powder diffraction pattern thereof represented by diffraction angle 2*θ* angle has characteristic peaks at 4.213, 6.650, 8.491, 9.593, 10.897, 12.831, 13.464, 13.954, 14.943, 15.666, 16.616, 17.259, 18.161, 19.245, 24.822, 25.665 and 26.618, wherein the error range of the 2*θ* angle is ±0.20.

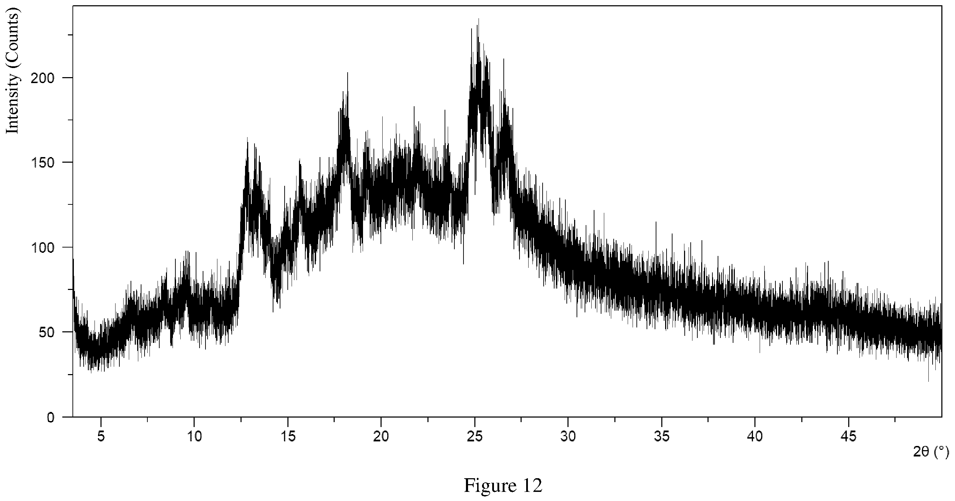

In some embodiments, provided is the crystal form D of phosphate salt of the compound of formula I, wherein the X-ray powder diffraction pattern thereof represented by diffraction angle 2*θ* angle is as shown in Figure 12.

In some embodiments, provided is the crystal form D of phosphate salt of the compound of formula I, wherein the DSC spectrum thereof has endothermic peaks at 130°C and 143°C, with the error range of ±2°C.

The present disclosure also provides crystal form E of L-tartrate salt of the compound of formula I above, wherein the X-ray powder diffraction pattern thereof represented by diffraction angle 2*θ* angle has characteristic peaks at 6.383, 9.081, 12.936, 16.161 and 18.397, wherein the error range of the 2*θ* angle is ±0.20.

In some embodiments, provided is the crystal form E of L-tartrate salt of the compound of formula I, wherein the X-ray powder diffraction pattern thereof represented by diffraction angle 2*θ* angle has characteristic peaks at 6.383, 9.081, 12.936, 14.400, 16.161, 18.397 and 19.489, wherein the error range of the 2*θ* angle is ±0.20.

In some embodiments, provided is the crystal form E of L-tartrate salt of the compound of formula I, wherein the X-ray powder diffraction pattern thereof represented by diffraction angle 2*θ* angle has characteristic peaks at 6.383, 9.081, 12.936, 14.400, 16.161, 18.397, 19.489, 23.786, 24.536 and 26.203, wherein the error range of the 2*θ* angle is ±0.20.

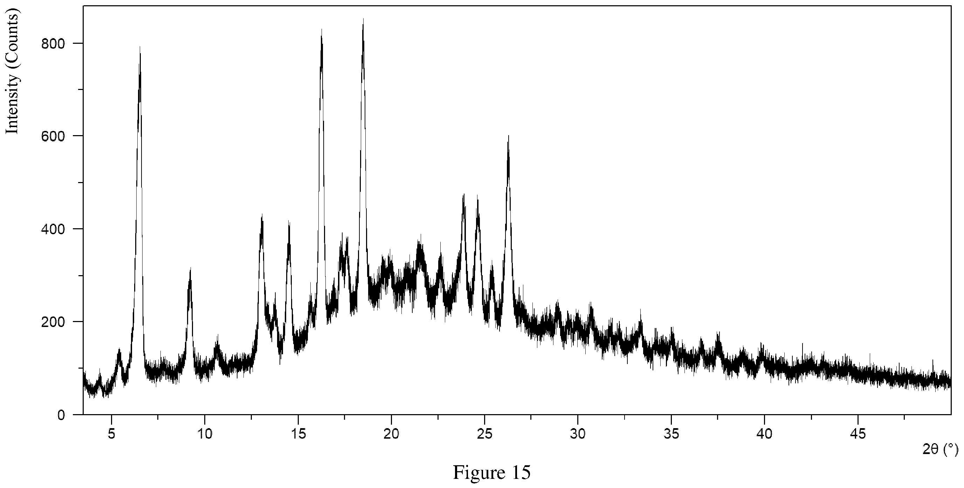

In some embodiments, provided is the crystal form E of L-tartrate salt of the compound of formula I, wherein the X-ray powder diffraction pattern thereof represented by diffraction angle 2*θ* angle is as shown in Figure 15.

In some embodiments, provided is the crystal form E of L-tartrate salt of the compound of formula I, wherein the DSC spectrum thereof has an endothermic peak at 132°C, with the error range of ±2°C.

The present disclosure also provides crystal form F of hydrobromide salt of the compound of formula I above, wherein the X-ray powder diffraction pattern thereof represented by diffraction angle 2*θ* angle has characteristic peaks at 7.133, 12.485, 14.422, 17.721 and 18.823, wherein the error range of the 2*θ* angle is ±0.20.

In some embodiments, provided is the crystal form F of hydrobromide salt of the compound of formula I, wherein the X-ray powder diffraction pattern thereof represented by diffraction angle 2*θ* angle has characteristic peaks at 6.152, 7.133, 10.827, 12.485, 14.422, 17.721 and 18.823, wherein the error range of the 2*θ* angle is ±0.20.

In some embodiments, provided is the crystal form F of hydrobromide salt of the compound of formula I, wherein the X-ray powder diffraction pattern thereof represented by diffraction angle 2*θ* angle has characteristic peaks at 6.152, 7.133, 10.827, 12.485, 14.422, 17.721, 18.823, 20.738, 21.656, 22.309, 23.238, 25.116, 25.672 and 27.416, wherein the error range of the 2*θ* angle is ±0.20.

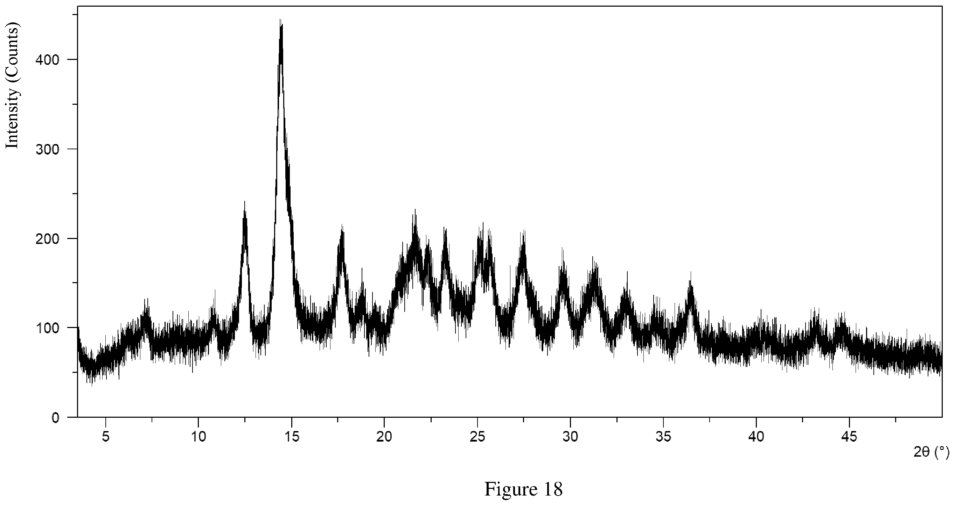

In some embodiments, provided is the crystal form F of hydrobromide salt of the compound of formula I, wherein the X-ray powder diffraction pattern thereof represented by diffraction angle 2*θ* angle is as shown in Figure 18.

In some embodiments, provided is the crystal form F of hydrobromide salt of the compound of formula I, wherein the DSC spectrum thereof has endothermic peaks at 99°C and 216°C, with the error range of ±2°C.

The term "2*θ* or 2*θ* angle" in the present disclosure refers to a diffraction angle, *θ* being the Bragg angle, and the unit of which is ° or degree. The error range of 2*θ* of each characteristic peak is ±0.20 (including the case in which a number with more than one decimal is rounded), and specifically, -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, and 0.20.

The present disclosure also provides a pharmaceutical composition, comprising the pharmaceutically acceptable salt of the compound of formula I above, crystal form A of hydrochloride salt of the compound of formula I, crystal form B of methanesulfonate salt of the compound of formula I, crystal form C of methanesulfonate salt of the compound of formula I, crystal form D of phosphate salt of the compound of formula I, crystal form E of L-tartrate salt of the compound of formula I or crystal form F of hydrobromide salt of the compound of formula I, and a pharmaceutically acceptable excipient.

The present disclosure also provides a method for preparing a pharmaceutical composition, comprising a step of mixing crystal form A of hydrochloride salt of the compound of formula I, crystal form B of methanesulfonate salt of the compound of formula I, crystal form C of methanesulfonate salt of the compound of formula I, crystal form D of phosphate salt of the compound of formula I, crystal form E of L-tartrate salt of the compound of formula I or crystal form F of hydrobromide salt of the compound of formula I with a pharmaceutically acceptable excipient.

The present disclosure also relates to use of the pharmaceutically acceptable salt of the compound of formula I above, crystal form A of hydrochloride salt of the compound of formula I, crystal form B of methanesulfonate salt of the compound of formula I, crystal form C of methanesulfonate salt of the compound of formula I, crystal form D of phosphate salt of the compound of formula I, crystal form E of L-tartrate salt of the compound of formula I, crystal form F of hydrobromide salt of the compound of formula I or the above composition, in the preparation of a medicament for preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa,, or cancer.

The present disclosure also relates to a method for preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa,, or cancer, comprising administering a therapeutically effective amount of the pharmaceutically acceptable salt of the compound of formula I above, crystal form A of hydrochloride salt of the compound of formula I, crystal form B of methanesulfonate salt of the compound of formula I, crystal form C of methanesulfonate salt of the compound of formula I, crystal form D of phosphate salt of the compound of formula I, crystal form E of L-tartrate salt of the compound of formula I, crystal form F of hydrobromide salt of the compound of formula I or the above composition, to a subject in need thereof.

The present disclosure also relates to the pharmaceutically acceptable salt of the compound of formula I above, crystal form A of hydrochloride salt of the compound of formula I, crystal form B of methanesulfonate salt of the compound of formula I, crystal form C of methanesulfonate salt of the compound of formula I, crystal form D of phosphate salt of the compound of formula I, crystal form E of L-tartrate salt of the compound of formula I, crystal form F of hydrobromide salt of the compound of formula I or the above composition, for use in preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa,, or cancer.

The term "excipient" in the present disclosure includes, but is not limited to, any adjuvant, carrier, glidant, sweetener, diluent, preservative, dye/colorant, flavor enhancer, surfactant, wetting agent, dispersant, suspending agent, stabilizer, isotonic agent or emulsifier that has been approved by the U.S. Food and Drug Administration and is acceptable for use in humans or livestock animals.

The starting material used in the method for preparing the crystal form of the present disclosure can be a compound in any form, and the specific form includes, but is not limited to, amorphous form, arbitrary crystal form, hydrate, solvate and the like.

The term "differential scanning calorimetry or DSC" in the present disclosure means to measure the temperature difference and heat flow difference between the sample and the reference during the heating or constant temperature process of the sample, to characterize all physical and chemical changes associated with the thermal effect, and to obtain phase change information of the sample.

In the present disclosure, the values, such as those related to the determination and calculation of substance contents, inevitably have a degree of error. In general, ±10% belongs to a reasonable range of error. There is a degree of error depending on the context in which it is used, and the error is no more than ±10% and may be ±9%, ±8%, ±7%, ±6%, ±5%, ±4%, ±3%, ±2% or ±1%, preferably ±5%.

### DESCRIPTION OF THE DRAWINGS

Figure 1 is the X-ray powder diffraction (XRPD) pattern of crystal form A of hydrochloride salt of the compound of formula I;
Figure 2 is the DSC spectrum of crystal form A of hydrochloride salt of the compound of formula I;
Figure 3 is the thermogravimetric analysis (TGA) spectrum of crystal form A of hydrochloride salt of the compound of formula I;
Figure 4 is the dynamic vapor sorption (DVS) plot of crystal form A of hydrochloride salt of the compound of formula I;
Figure 5 shows XRPD patterns of the crystal form before and after DVS, in which the upper XRPD pattern is one before DVS and the lower XRPD pattern is one after DVS;
Figure 6 is an XRPD pattern of crystal form B of methanesulfonate salt of the compound of formula I;
Figure 7 is the DSC spectrum of crystal form B of methanesulfonate salt of the compound of formula I;
Figure 8 is the TGA spectrum of crystal form B of methanesulfonate salt of the compound of formula I;
Figure 9 is an XRPD pattern of crystal form C of methanesulfonate salt of the compound of formula I;
Figure 10 is the DSC spectrum of crystal form C of methanesulfonate salt of the compound of formula I;
Figure 11 is the TGA spectrum of crystal form C of methanesulfonate salt of the compound of formula I;
Figure 12 is the XRPD pattern of crystal form D of phosphate salt of the compound of formula I;
Figure 13 is the DSC spectrum of crystal form D of phosphate salt of the compound of formula I;
Figure 14 is the TGA spectrum of crystal form D of phosphate salt of the compound of formula I;
Figure 15 is the XRPD pattern of crystal form E of L-tartrate salt of the compound of formula I;
Figure 16 is the DSC spectrum of crystal form E of L-tartrate salt of the compound of formula I;
Figure 17 is the TGA spectrum of crystal form E of L-tartrate salt of the compound of formula I;
Figure 18 is the XRPD pattern of crystal form F of hydrobromide salt of the compound of formula I;
Figure 19 is the DSC spectrum of crystal form F of hydrobromide salt of the compound of formula I;
Figure 20 is the TGA spectrum of crystal form F of hydrobromide salt of the compound of formula I;
Figure 21 is the nuclear magnetic resonance (NMR) spectrum of crystal form F of hydrobromide salt of the compound of formula I;
Figure 22 is the XRPD pattern of crystal form A' of hydrochloride salt of the compound of formula I;
Figure 23 is the DSC spectrum of crystal form A' of hydrochloride salt of the compound of formula I;
Figure 24 is the TGA spectrum of crystal form A' of hydrochloride salt of the compound of formula I.

### DETAILED DESCRIPTION OF THE INVENTION

The present disclosure will be explained in more detail through examples or experimental examples below. The examples or experimental examples of the present disclosure are merely intended to describe the technical solutions of the present disclosure, and should not be considered as limiting the spirit and scope of the present disclosure.

The abbreviations used in the present disclosure are explained as follows:
- XRPD: X-ray powder diffraction
- DSC: Differential scanning calorimetry
- TGA: Thermogravimetric analysis
- DVS: Dynamic vapor sorption
- ¹H-NMR: Liquid nuclear magnetic resonance hydrogen spectroscopy
- DMF: N,N-dimethylformamide
- MEK: Butanone
- MTBE: Methyl tert-butyl ether
- THF: Tetrahydrofuran
- IPA: Isopropanol
- ACN: Acetonitrile
- MeOH: Methanol
- EOH: Ethanol
- ACT: Acetone
- PA: Phosphoric acid
- TA: Tartaric acid
- HBr: Hydrobromic acid
- HCl: Hydrochloric acid
- DCM: Dichloromethane

Test conditions for the instruments used in experiments of the present disclosure:
1. X-ray Powder Diffraction, XRPD
Instrument type: Malver Panalytical Aeris X-ray powder diffractometer
Ray: monochromatic Cu-Kα ray (λ=1.54188)
Scanning mode: θ/2θ, Scanning range (2θ range) : 3.5-50°
Voltage: 40 kV, Electric current: 15 mA
2. Differential Scanning Calorimetry, DSC
Instrument type: TA DSC250
Purge gas: Nitrogen; Nitrogen purge rate: 50 mL/min
Heating rate: 10°C/min
Temperature range: 25°C-250°C
3. Thermogravimetric Analysis, TGA
Instrument type: TA TGA550
Purge gas: Nitrogen; Nitrogen purge rate: 20 ml/min
Scanning rate: 10°C/min
Temperature range: 30°C-350°C
4. Dynamic vapor sorption (DVS)

Using SMS Intrinsic PLUS, the detection is performed at 25°C, with humidity change of 50% -0% -90% with a step size of 10%, in which the criteria for judging is that the mass change dM/dT for each gradient is less than 0.002%, TMAX is 360 min, and two cycles are performed.

5. High performance liquid chromatography (HPLC) described in the stability test of the crystal form A of the present disclosure is determined on Agilent 1260 Infinity II.

In the content detection method by the high performance liquid chromatography described in the present disclosure, HPLC conditions are as follows: chromatographic column, Agilent Eclipse Plus C18 4.6mm*150mm,3.5µm; mobile phase, A - 10 mmol/L solution of sodium dihydrogen phosphate (pH 8.0), and B - ACN; flow rate, 1.0 ml/min; wavelength, 210 nm.

In the related substances detection method by the high performance liquid chromatography described in the present disclosure, HPLC conditions are as follows: chromatographic column, Agilent Eclipse Plus C18 4.6mm*150mm,3.5µm; mobile phase, A - 10 mmol/L solution of ammonium acetate, and B - ACN; flow rate, 1.0 ml/min; wavelength, 210 nm.

### Example 1: Preparation of (S)-N-((5)-1-cyano-2-(8-cyano-2-fluoro-6H-benzo[c]chromen-3-yl)ethyl)-1,4-oxazepane-2-carboxamide (the compound of formula I)

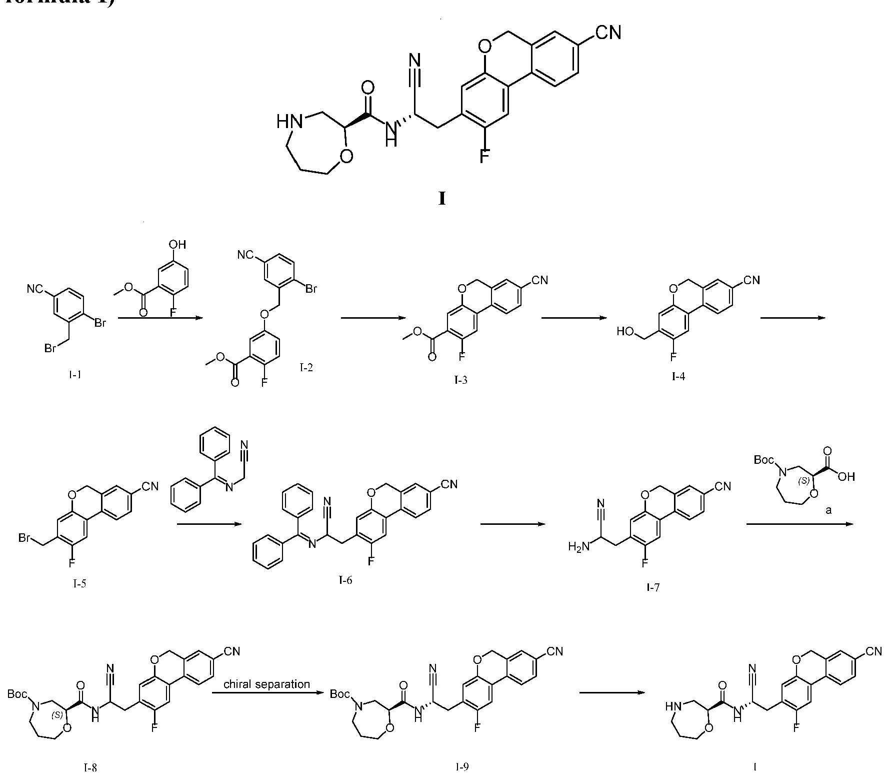

### Synthesis of Compound I-2

Methyl 2-fluoro-5-hydroxybenzoate (4.30 g, 25.29 mmol), 4-bromo-3-(bromomethyl)benzonitrile (Compound **I-1)** (7.72 g, 25.30 mmol) and potassium carbonate (6.99 g, 50.58 mmol) were dissolved in *N,N*-dimethylformamide (50 mL) at room temperature, and the reaction mixture was heated to 40°C and stirred for 12 hours. Water (300 mL) was added thereto, and the mixture was extracted with ethyl acetate (100 mL×2). The organic phases were combined, washed with saturated brine (100 mL), dried over anhydrous sodium sulfateand filtered. The filtrate was concentrated under reduced pressure, and the resulting residue was purified by column chromatography (SiO₂, petroleum ether/ethyl acetate=9/1) to obtain Compound **I-2.** ¹H NMR (400 MHz, CDCl₃) δ 7.87 (d, 1H), 7.73 (d, 1H), 7.55-7.48 (m, 2H), 7.18-7.09 (m, 2H), 5.11 (s, 2H), 3.95 (s, 3H).

### Synthesis of Compound I-3

Compound **I-2** (2.90 g, 6.37 mmol), palladium acetate (0.14 g, 0.64 mmol), potassium carbonate (1.76 g, 12.73 mmol) and tricyclohexylphosphine tetrafluoroborate (0.23 g, 0.64 mmol) were dissolved in N,N-dimethylformamide (30 mL) at room temperature under nitrogen atmosphere, and the reaction mixture was heated to 120°C and stirred for 1.5 hours. After the reaction was complete, the resulting mixture was cooled to room temperature, followed by the addition of water (200 mL), and extracted with ethyl acetate (200 mL×2). The organic phases were combined, washed with saturated brine (200 mL), dried over anhydrous sodium sulfateand filtered. The filtrate was concentrated under reduced pressure, and the resulting residue was purified by column chromatography (SiO₂, petroleum ether/ethyl acetate=4/1) to obtain Compound **1-3.** ¹H NMR (400 MHz, CDCl₃) δ 7.80-7.72 (m, 2H), 7.60 (d, 1H), 7.52-7.49 (m, 2H), 5.18 (s, 2H), 3.98 (s, 3H).

### Synthesis of Compound I-4

Compound **I-3** (1.20 g, 3.81 mmol) and lithium borohydride (0.25 g, 11.47 mmol) were dissolved in tetrahydrofuran (25 mL) at room temperature, and the reaction mixture was heated to 55°C and stirred for 2 hours. After the reaction was complete, the reaction mixture was cooled to room temperature, followed by the addition of water (200 mL), and extracted with ethyl acetate (200 mL×2). The organic phases were combined, washed with saturated brine (200 mL), dried over anhydrous sodium sulfateand filtered. The filtrate was concentrated under reduced pressure, and the resulting residue was purified by column chromatography (SiO₂, petroleum ether/ethyl acetate=9/1) to obtain Compound **I-4.** ¹H NMR (400 MHz, CDCl₃) δ 7.67 (s, 2H), 7.47 (s, 1H), 7.38 (d, 1H), 7.12 (d, 1H), 5.11 (s, 2H), 4.77 (s, 2H).

### Synthesis of Compound I-5

Compound **I-3** (450 mg, 1.59 mmol) was dissolved in dichloromethane (15 mL) and then phosphorus tribromide (520 mg, 1.92 mmol) was added dropwise thereto at room temperature, and the reaction mixture was stirred for 20 minutes at room temperature. After the reaction was complete, water (100 mL) was added thereto, and the resulting mixture was extracted with ethyl acetate (100 mL×2). The organic phases were combined, washed with saturated brine (100 mL), dried over anhydrous sodium sulfateand filtered. The filtrate was concentrated under reduced pressure, and the resulting residue was purified by column chromatography (SiO₂, petroleum ether/ethyl acetate=9/1) to obtain Compound **I-5.** ¹H NMR (300 MHz, CDCl₃) δ 7.73 (s, 2H), 7.52 (s, 1H), 7.45 (d, 1H), 7.10 (d, 1H), 5.17 (s, 2H), 4.53 (s, 2H).

### Synthesis of Compound I-6

N-(diphenylmethylene)aminoacetonitrile (250 mg, 1.14 mmol), Compound **I-5** (470 mg, 1.26 mmol), benzyltrimethylammonium chloride (22 mg, 0.12 mmol) and sodium hydroxide (91 mg, 2.3 mmol) were dissolved in a mixed solvent of dichloromethane (6 mL) and water (6 mL) at room temperature, and the reaction mixture was heated to 35°C and stirred for 24 hours. Water (30 mL) was added thereto, and the resulting mixture was extracted with dichloromethane (30 mL×2). The organic phases were combined, washed with saturated brine (20 mL), dried over anhydrous sodium sulfateand filtered. The filtrate was concentrated under reduced pressure, and the resulting residue was purified by column chromatography (SiO₂, petroleum ether/ethyl acetate=4/1) to obtain Compound **1-6.** MS-ESI: *m*/*z* 458.4 [M+1]⁺.

### Synthesis of Compound I-7

Compound **I-6** (520 mg, 0.90 mmol) was dissolved in tetrahydrofuran (10 mL) and then 1 M aqueous hydrochloric acid solution (4 mL) was added dropwise thereto at room temperature, and the reaction mixture was stirred for 1 hour at room temperature. After the reaction was complete, saturated aqueous sodium bicarbonate solution (30 mL) was added thereto, and the resulting mixture was extracted with ethyl acetate (30 mL×3). The organic phases were combined, washed with saturated brine (30 mL), dried over anhydrous sodium sulfateand filtered. The filtrate was concentrated under reduced pressure, and the resulting residue was purified by column chromatography (SiO₂, petroleum ether/ethyl acetate=2/1) to obtain Compound **I-7.** MS-ESI: *m*/*z* 293.9 [M+1]⁺.

### Synthesis of Compound I-8

Compound **I-7** (220 mg, 0.68 mmol), Compound **a** (170 mg, 0.69 mmol), *N,N,N',N'-*tetramethyl-O-(7-azabenzotriazol-1-yl)uronium hexafluorophosphate (360 mg, 0.95 mmol) and *N,N*-diisopropylethylamine (250 mg, 1.93 mmol) were dissolved in *N,N-*dimethylformamide (5 mL) at room temperature, and the reaction mixture was stirred for 3 hours at room temperature. After the reaction was complete, the resulting mixture was purified by preparative liquid chromatography (C18, acetonitrile/water system) to obtain Compound **I-8.** MS-ESI: *m*/*z* 465.1 [M-56+1]⁺.

### Synthesis of Compound I-9

Compound **I-8** (280 mg, 0.51 mmol) was subjected to chiral resolution (column: chiralpak IE, 250*25 mm, 5 µm; mobile phase: n-hexane, ethanol; gradient: n-hexane phase 30%; flow rate: 15 mL/min; column temperature: 30°C) to obtain Compound **I-9** (two diastereomer peaks in total, Compound **I-9** being the first elution peak). ¹H NMR (300 MHz, DMSO-*d*₆) δ 8.89 (d, 1H), 8.08 (d, 1H), 7.94-7.89 (m, 2H), 7.83 (s, 1H), 7.07 (d, 1H), 5.24-5.14 (m, 2H), 5.09-5.07 (m, 1H), 4.15-4.12 (m, 1H), 3.99-3.87 (m, 2H), 3.63-3.56 (m, 2H), 3.29-3.02 (m, 4H), 1.89-1.79 (m, 2H), 1.41-1.36 (m, 9H).

### Synthesis of Compound I

Compound **I-9** (85 mg, 0.16 mmol) was dissolved in formic acid (1 mL) at room temperature, and the reaction mixture was heated to 40°C and stirred for 1 hours. After the reaction was complete, the resulting mixture was purified by preparative liquid chromatography (C18, ammonium bicarbonate/acetonitrile/water system) to obtain Compound **I.** The resulting compound was determined as being amorphous by XRPD. MS-ESI: m/z 421.2 [M+1]⁺. ¹H NMR (400 MHz, DMSO-d6) δ 8.69 (d, 1H), 8.05 (d, 1H), 7.89-7.86 (m, 2H), 7.80 (s, 1H), 7.03 (d, 1H), 5.20-5.12 (m, 2H), 5.09-5.02 (m, 1H), 4.00-3.97 (m, 1H), 3.88-3.83 (m, 1H), 3.77-3.68 (m, 1H), 3.25-3.14 (m, 2H), 3.05-2.98 (m, 1H), 2.82-2.72 (m, 1H), 2.62-2.53 (m, 2H) , 1.80-1.66 (m, 2H).

### Example 2: Biological evaluation of the compound of formula I - In vitro CatC cell activity experiment

### 1. Experimental materials

| **Name** | **Brand** | **Cat No./Model** |
|---|---|---|
| U937 | ATCC | CRL-1593.2 |
| H-Gly-Phe-AMC | Lai'ang | P201221-K3 |
| AZD7986 (alias: INS 1007) | MedChemExpress | HY-101056 |
| OptiPlate^{™}-384 F black-bottom analysis plate | PerkinElmer | 6007279 |
| RPMI1640 culture medium +10%FBS | Gbico | |

### 2. Experimental steps

Complete culture medium RPMI 1640+10% FBS was formulated and mixed well. The U937 cell line was resuscitated and passaged for about two passages, and the cell strains with good growth status were selected. The cell suspension was transferred into a centrifuge tube, and centrifuged at 800 to 1000 rpm for 3 to 5 minutes. The supernatant was discarded. An appropriate volume of culture medium was added to the centrifuge tube, which was gently pipetted to resuspend the cells evenly. The cells were counted using a Vi-Cell XR cell counter. The cell suspension was adjusted to an appropriate concentration. The cell suspension was added into a 384-well plate, at 25000 µL/well. The compound was formulated into a 10 mM solution with DMSO, diluted with DMSO to obtain a 1 mM solution, and subjected to a semilogarithmic dilution by HPD300 with DMSO to obtain 10 concentrations. Gly-Phe-AFC was formulated into a 35 mM solution with DMSO and divided. Gly-Phe-AFC was formulated to 1.75 mM with serum-free culture medium. After adding the compounds and incubating for 1 hour in an incubator, the substrate-AFC was added (12.5 µL. was added to the plate). The plate was incubated for 30 minutes, followed by measurement. The plate was placed and read in EnSpire, and the fluorescence values were recorded at Ex 400 nm and Em 505 nm. The inhibition rate was calculated according to the following formula: inhibition rate (%) = (1 - (RFU compound - RFU blank)/(RFU DMSO - RFU blank)) × 100%. XLFit was used to draw the drug efficacy inhibition rate curve and calculate the IC₅₀ value. 4-parameter model [fit = (A + ((B - A)/ (1 + ((C/x) ^D))))] was used.

The *in vitro* inhibition of the compound of formula I of the present disclosure on CatC cell activity was determined through the above test, and the determined IC₅₀ value was 6.1 nM, indicating that the inhibitory effect on CatC cell activity was significant.

### Example 3: Preparation of crystal form A of hydrochloride salt of the compound of formula I

### Method 1:

The compound of formula I (1.01 g) was added to 76 mL of acetone and stirred at 60°C, and after the solution was clear, 586.0 µL. of diluted hydrochloric acid solution (prepared by measuring 500 µL. of hydrochloric acid and 500 µL of purified water and mixing them well for use) was added thereto. After adding hydrochloric acid dropwise, a precipitation phenomenon occurred. The heating was stopped and the mixture was stirred overnight. The next day, the sample was poured onto a filter paper, subjected to air drying in a fume hood, and collected to obtain hydrochloride salt of the compound of formula I, 960 mg (off white solid, yield: 95%), which was stored hermetically.

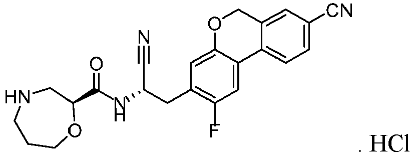

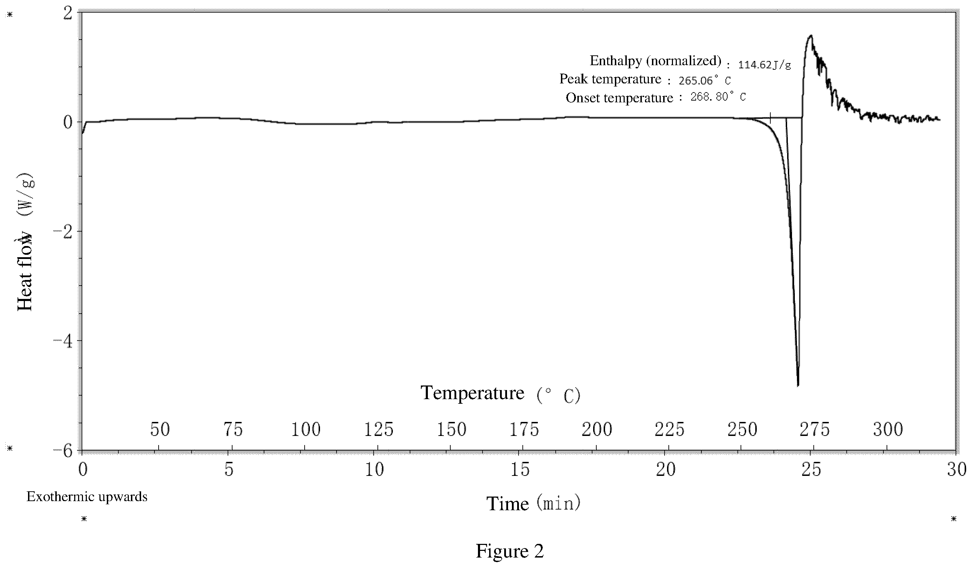

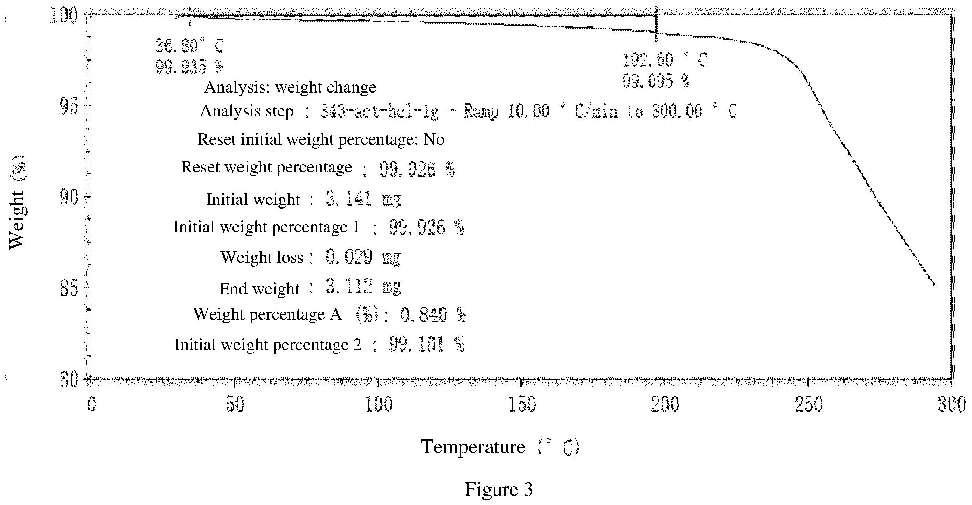

The product was detected by the X-ray powder diffraction and defined as crystal form A of hydrochloride salt of the compound of formula I. The XRPD pattern is shown in Figure 1, and the positions of the characteristic peaks are shown in Table 1. The DSC spectrum, as shown in Figure 2, has an endothermic peak at about 268 °C. The TGA spectrum, as shown in Figure 3, shows a rapid weight loss at 200°C or higher.

The chloride ion content of the sample of crystal form A was determined by ion chromatography. The result was 7.64% substantially consistent with the theoretical value of 7.7% for 1 mol hydrochloride salt, confirming a salt-forming with hydrochloric acid and free base of the compound of formula I in a molar ratio of 1:1.

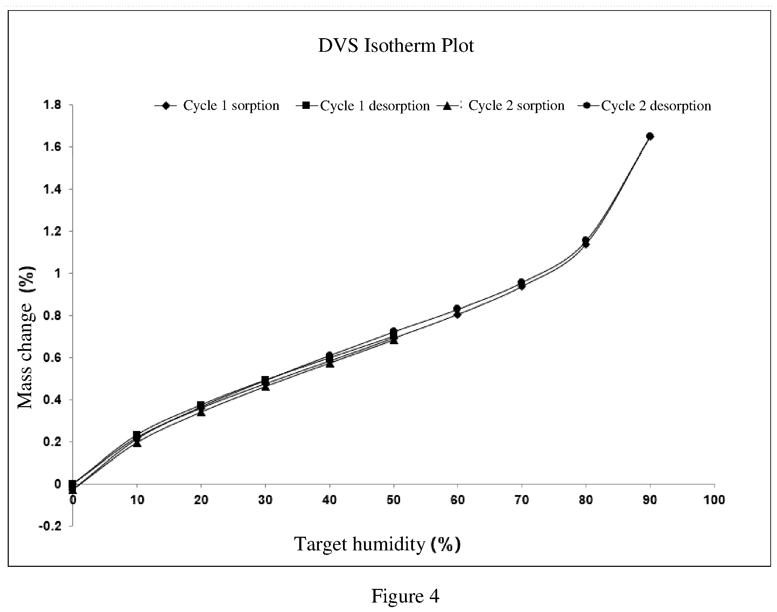

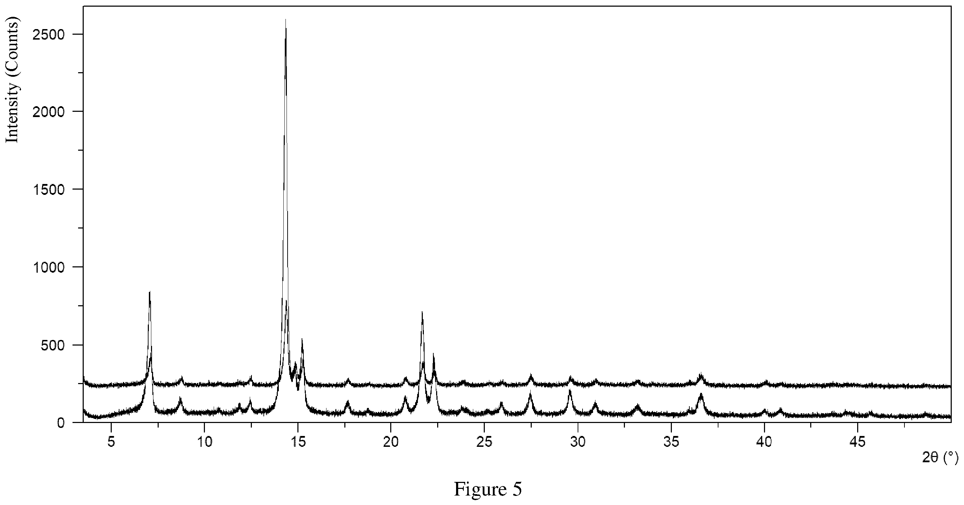

The DVS detection, as shown in Figure 4, shows that the weight gain by hygroscopicity is approximately 1.1% under accelerated experimental conditions (i.e., 80% RH). During the humidity change of 0% -90% RH, the desorption process of the sample coincides with the adsorption process thereof. After DVS detection, the crystal form was redetermined, and the XRPD pattern is shown in Figure 5, showing that the crystal form did not change before and after DVS detection.

**Table 1. Data of XRPD characteristic diffraction peaks of crystal form A of hydrochloride salt**

| Peak No. | 2θ[° or degree] | d[Å] | Relative strength |
|---|---|---|---|
| 1 | 7.043 | 12.55103 | 28.56 |
| 2 | 8.719 | 10.14186 | 17.66 |
| 3 | 10.141 | 8.72255 | 7.11 |
| 4 | 10.737 | 8.24031 | 15.24 |
| 5 | 11.853 | 7.46629 | 6.13 |
| 6 | 12.430 | 7.12118 | 34.18 |
| 7 | 14.356 | 6.16987 | 100 |
| 8 | 14.840 | 5.96987 | 62.27 |
| 9 | 15.250 | 5.81023 | 34.01 |
| 10 | 17.686 | 5.01505 | 16.34 |
| 11 | 18.800 | 4.72034 | 7.13 |
| 12 | 19.367 | 4.58341 | 2.58 |
| 13 | 20.809 | 4.26878 | 14.15 |
| 14 | 21.697 | 4.09611 | 24.41 |
| 15 | 22.305 | 3.98579 | 17.48 |
| 16 | 23.242 | 3.82714 | 9.16 |
| 17 | 23.818 | 3.73588 | 9.23 |
| 18 | 24.821 | 3.58719 | 6.44 |
| 19 | 25.635 | 3.47513 | 15.1 |
| 20 | 27.433 | 3.25132 | 15.04 |
| 21 | 27.997 | 3.18704 | 2.81 |
| 22 | 29.607 | 3.01731 | 12.3 |
| 23 | 30.950 | 2.88938 | 5.12 |
| 24 | 31.342 | 2.85413 | 5.5 |
| 25 | 32.709 | 2.73788 | 1.75 |
| 26 | 33.127 | 2.70434 | 7.09 |
| 27 | 34.035 | 2.63422 | 2.2 |
| 28 | 34.597 | 2.59269 | 2.51 |
| 29 | 35.984 | 2.4959 | 6.05 |
| 30 | 36.565 | 2.45753 | 13.26 |
| 31 | 40.025 | 2.25274 | 4.15 |

### Method 2:

30 mg of free base of the compound of formula I was added to 0.3 mL of the solvent in Table 2 to obtain a suspension under stirring conditions. 4 mol/L aqueous hydrochloric acid solution was added to the suspension in a molar ratio of 1.2:1 to the free base, and the liquid volume was supplemented to 1.2 mL. The mixture was stirred for 3 hours at 50°C, then cooled to 25°C, and kept at this temperature under stirring overnight. The solid precipitated from the system was centrifuged, and dried under reduced pressure for 6 hours at 60°C to obtain a solid. The resulting solid was determined as crystal form A by XRPD detection.

**Table 2. Preparation of crystal form A of hydrochloride salt by trituration**

| Solvent | Crystal form | Solvent residue |
|---|---|---|
| Ethanol/water (volume ratio 8/2) | A | 0.6% Ethanol |
| Isopropanol/water (volume ratio 8/2) | A | 0.3% Isopropanol |
| Ethanol | A | 0.8 % |
| Ethyl acetate | A | 0.67 % |
| Acetonitrile | A | 1.24 % |
| Tetrahydrofuran | A | 0.68 % |
| Dimethyl sulfoxide/water (volume ratio 8/2) | A | 1.9% Dimethyl sulfoxide |
| 1,4-Dioxane | A | 1.1 % |

### Method 3:

10 mg of hydrochloride salt of the compound of formula I was added to 0.2 ml of the solvent as shown in Table 3, kept in a triturating state in the solvent, and stirred for one week. The solvent was quickly removed using a filter paper, and the resulting solid was determined as crystal form A by XRPD detection.

**Table 3. Preparation of crystal form A of hydrochloride salt by trituration**

| Solvent (molar ratio of water in the mixed solvent) | Crystal form | |
|---|---|---|
| | Stirring at room temperature | Stirring at 50°C |
| Water | A | A |
| Methanol | A | A |
| Ethanol | A | A |
| 0.14 (Water/ MeOH) | A | A |
| 0.26 (Water/ MeOH) | A | A |
| 0.37 (Water/ MeOH) | A | A |
| 0.47 (Water/ MeOH) | A | A |
| 0.57 (Water/ MeOH) | A | A |
| 0.66 (Water/ MeOH) | A | A |
| 0.74 (Water/ MeOH) | A | A |
| 0.82 (Water/ MeOH) | A | A |
| 0.90 (Water/ MeOH) | A | A |

### Example 4: Preparation of crystal form B of methanesulfonate salt of the compound of formula I

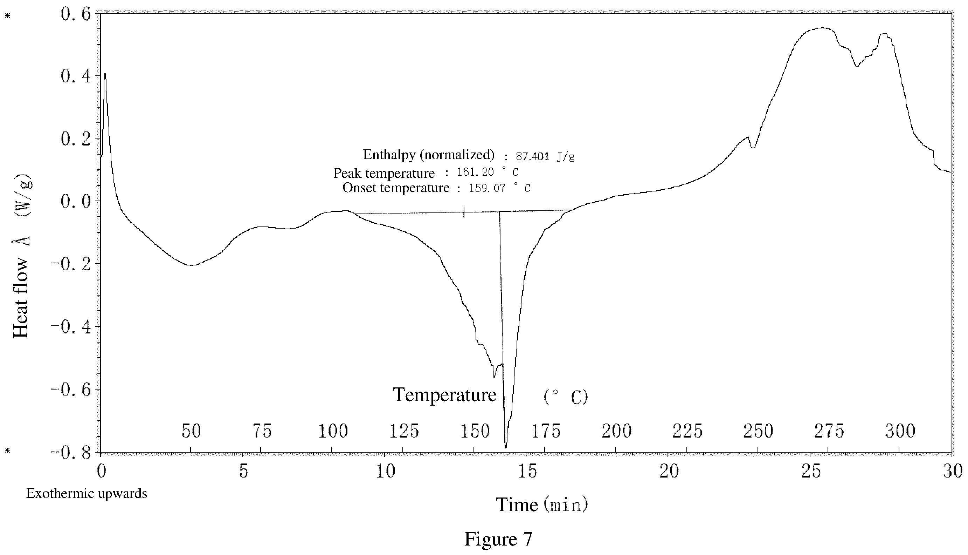

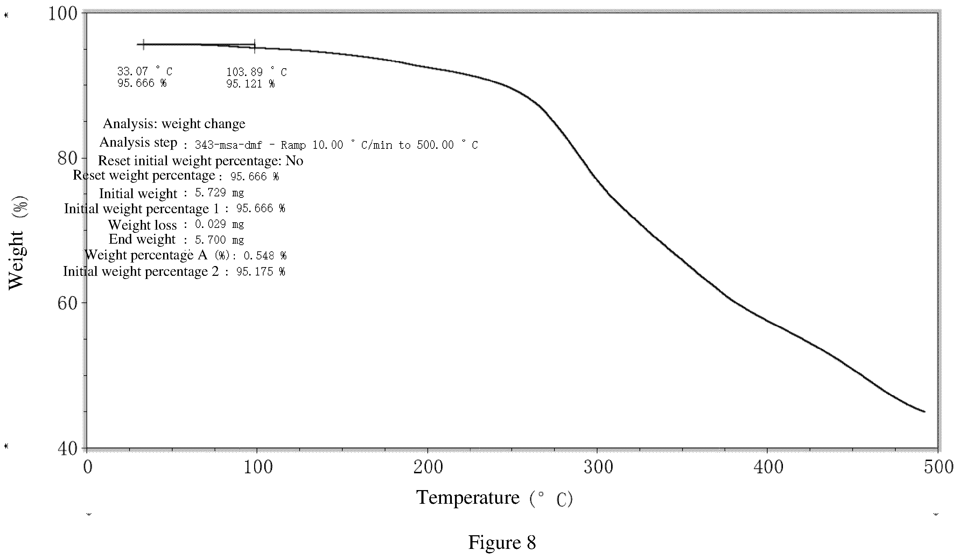

15 mg of the compound of formula I was dissolved in 1 ml of DMF, and methanesulfonic acid (4.1 mg) was added thereto. The solution was heated to 50°C and stirred for 4 hours at the constant temperature of 50°C, then cooled to 0°C and stirred for two days or longer at the controlled temperature to continue the reaction. After drying under vacuum, the solid was obtained as crystal form B of methanesulfonate salt of the compound of formula I. Its XRPD pattern is shown in Figure 6, and the positions of the characteristic peaks thereof are shown in Table 4. Its DSC spectrum is shown in Figure 7 with an endothermic peak at 161°C, showing low crystallinity. Its TGA spectrum is shown in Figure 8.

**Table 4. Data of XRPD characteristic diffraction peaks of crystal form B of methanesulfonate salt of the compound of formula I**

| Peak No. | 2θ[° or degree] | d[Å] | Relative strength |
|---|---|---|---|
| 1 | 6.717 | 13.15933 | 92.98 |
| 2 | 8.105 | 10.90849 | 15.46 |
| 3 | 8.783 | 10.06794 | 29.01 |
| 4 | 13.969 | 6.34008 | 20.09 |
| 5 | 15.902 | 5.57339 | 23.56 |
| 6 | 16.647 | 5.32545 | 100 |
| 7 | 17.515 | 5.06347 | 17.11 |
| 8 | 20.446 | 4.34386 | 84.22 |
| 9 | 21.069 | 4.21667 | 34.3 |
| 10 | 21.645 | 4.10575 | 33.59 |
| 11 | 23.549 | 3.77798 | 17.18 |
| 12 | 24.665 | 3.60954 | 45.1 |
| 13 | 26.284 | 3.39079 | 15.81 |
| 14 | 27.289 | 3.26817 | 16.14 |
| 15 | 27.667 | 3.22436 | 16.41 |

### Example 5: Preparation of crystal form C of methanesulfonate salt of the compound of formula I

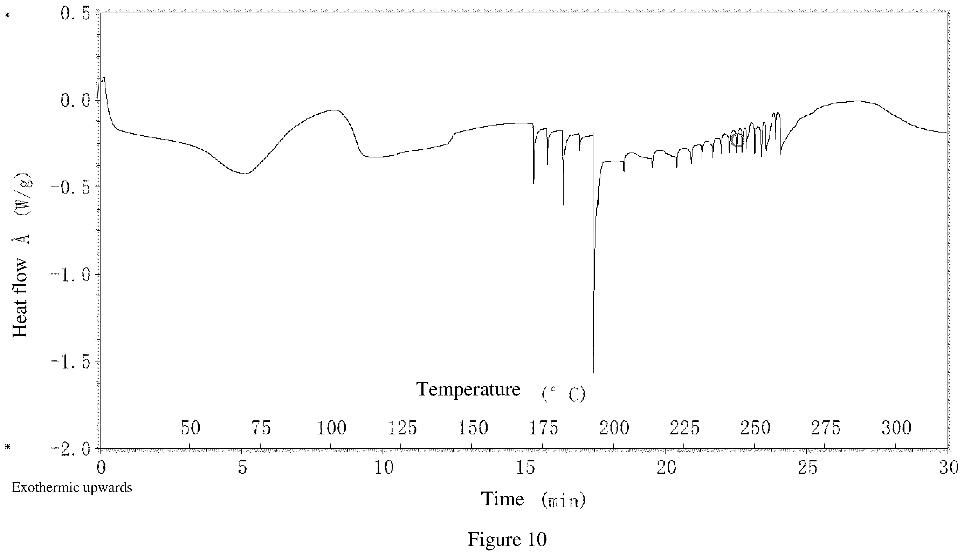

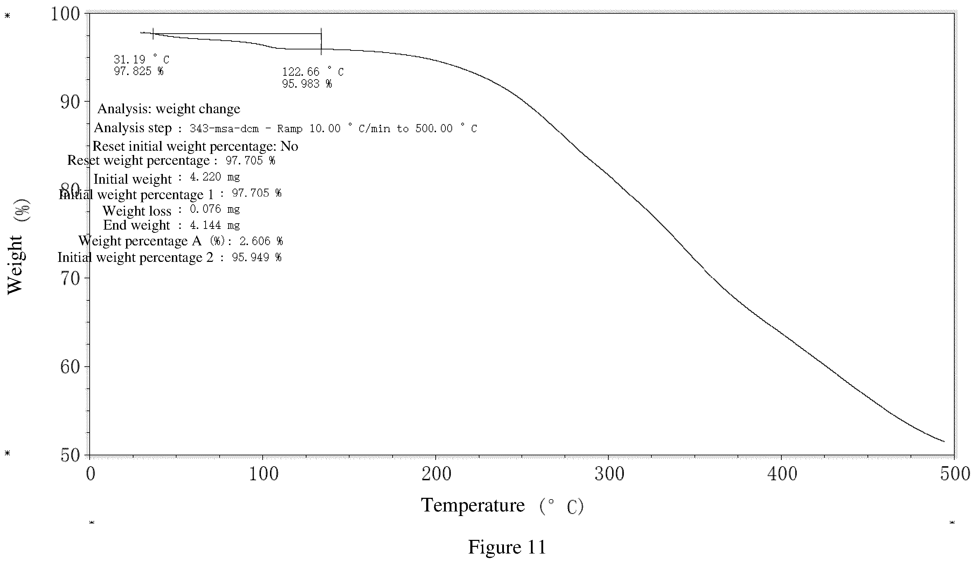

15 mg of the compound of formula I was dissolved in 1 ml of DCM, and methanesulfonic acid (4.1 mg) was added thereto. The solution was heated to 50°C and stirred for 4 hours at the constant temperature of 50°C, then cooled to 0°C and stirred for two days or longer at the controlled temperature to continue the reaction. After drying under vacuum, the solid obtained was determined as crystal form C of methanesulfonate salt of the compound of formula I by XRPD. The XRPD detection data of crystal form C of methanesulfonate salt of the compound of formula I are shown in Table 5, and its XRPD pattern is shown in Figure 9. Its DSC spectrum is shown in Figure 10 with an endothermic peak at about 194°C. Its TGA spectrum is shown in Figure 11.

**Table 5. Data of XRPD characteristic diffraction peaks of crystal form C of methanesulfonate salt**

| Peak No. | 2θ[° or degree] | d[Å] | Relative strength |
|---|---|---|---|
| 1 | 4.522 | 19.54332 | 7.11 |
| 2 | 7.747 | 11.41247 | 100 |
| 3 | 11.163 | 7.92629 | 13.18 |
| 4 | 12.676 | 6.98365 | 13.63 |
| 5 | 15.268 | 5.80348 | 41.95 |
| 6 | 16.824 | 5.26977 | 2.05 |
| 7 | 18.549 | 4.78362 | 25.17 |
| 8 | 19.759 | 4.49319 | 14.62 |
| 9 | 21.460 | 4.14088 | 18.59 |
| 10 | 22.539 | 3.94488 | 6.44 |
| 11 | 24.637 | 3.61358 | 12.28 |
| 12 | 25.497 | 3.49358 | 20.62 |
| 13 | 27.962 | 3.19099 | 4.11 |
| 14 | 28.876 | 3.09204 | 8.06 |

### Example 6: Preparation of crystal form D of phosphate salt of the compound of formula I

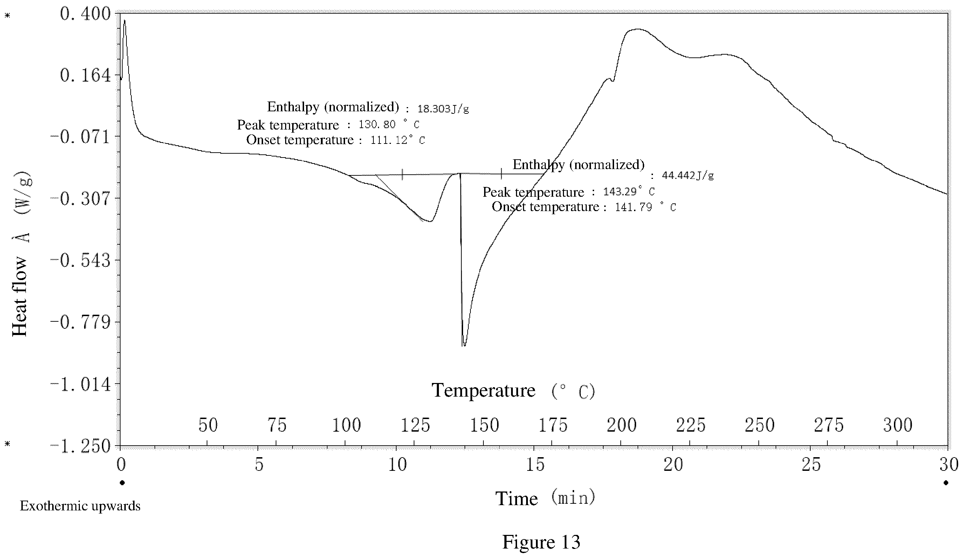

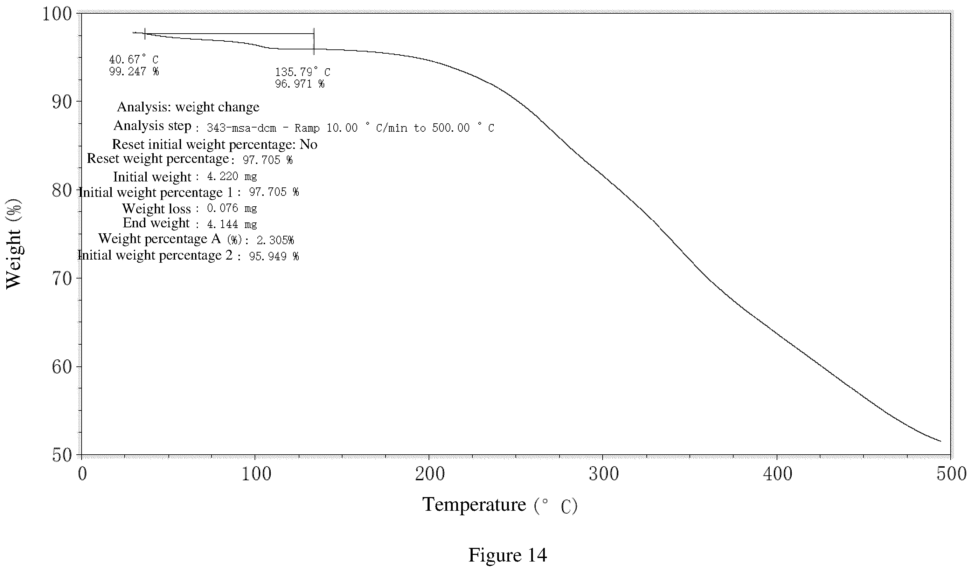

15 mg of the compound of formula I was dissolved in 1 ml of DCM, and phosphoric acid (4.2 mg) was added thereto. The solution was heated to 50°C and stirred for 4 hours at the constant temperature of 50°C, then cooled to 0°C and stirred for two days or longer at the controlled temperature to continue the reaction. After drying under vacuum, the solid obtained was determined as crystal form D of phosphate salt of the compound of formula I by XRPD. The XRPD detection data of crystal form D of phosphate salt of the compound of formula I are shown in Table 6, and its XRPD pattern is shown in Figure 12. Its DSC spectrum is shown in Figure 13 with endothermic peaks at 130°C and 143°C. Its TGA spectrum is shown in Figure 14. The result of ion chromatography detection showed the phosphate group content was 24.18%, indicating a salt-forming with phosphoric acid and free base in a ratio of approximately 1:1.

**Table 6. Data of XRPD characteristic diffraction peaks of crystal form D of phosphate salt of the compound of formula I**

| Peak No. | 2θ[° or degree] | d[Å] | Relative strength |
|---|---|---|---|
| 1 | 4.213 | 20.97159 | 4.17 |
| 2 | 6.650 | 13.29155 | 8.25 |
| 3 | 8.491 | 10.41392 | 14.89 |
| 4 | 9.593 | 9.22006 | 24.9 |
| 5 | 10.897 | 8.11964 | 7.24 |
| 6 | 12.831 | 6.89927 | 100 |
| 7 | 13.464 | 6.57653 | 76.67 |
| 8 | 13.954 | 6.3466 | 32.12 |
| 9 | 14.943 | 5.92898 | 13.21 |
| 10 | 15.666 | 5.65672 | 40.5 |
| 11 | 16.616 | 5.33535 | 23.6 |
| 12 | 17.259 | 5.13811 | 31.23 |
| 13 | 18.161 | 4.88477 | 63.97 |
| 14 | 19.245 | 4.61215 | 42.33 |
| 15 | 20.242 | 4.38722 | 18.39 |
| 16 | 20.854 | 4.2598 | 26.8 |
| 17 | 21.213 | 4.18848 | 23.16 |
| 18 | 22.022 | 4.03645 | 30.97 |
| 19 | 23.570 | 3.77463 | 24.99 |
| 20 | 24.822 | 3.58711 | 65.21 |
| 21 | 25.665 | 3.47109 | 83.4 |
| 22 | 26.618 | 3.349 | 54.82 |
| 23 | 27.818 | 3.2071 | 19.06 |
| 24 | 29.016 | 3.07746 | 11.27 |

### Example 7: Preparation of crystal form E of L-tartrate salt of the compound of formula I

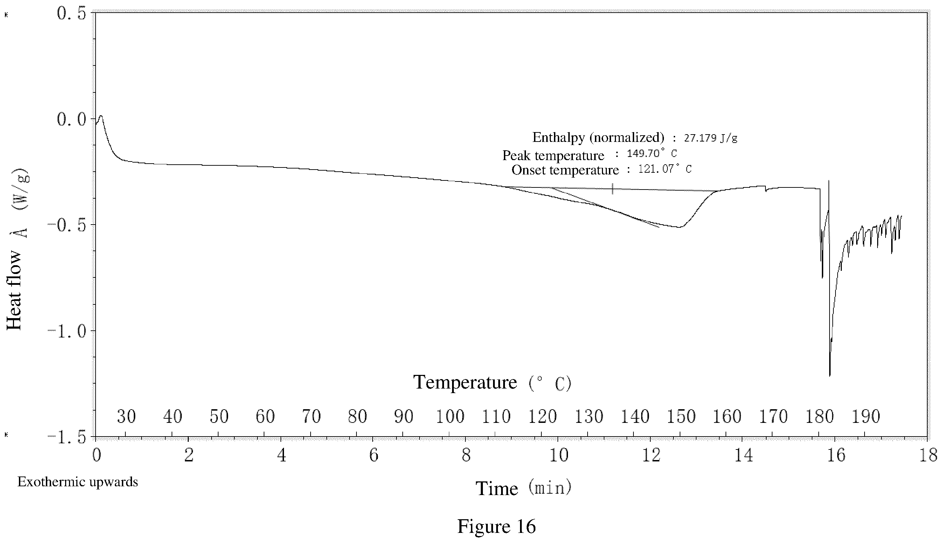

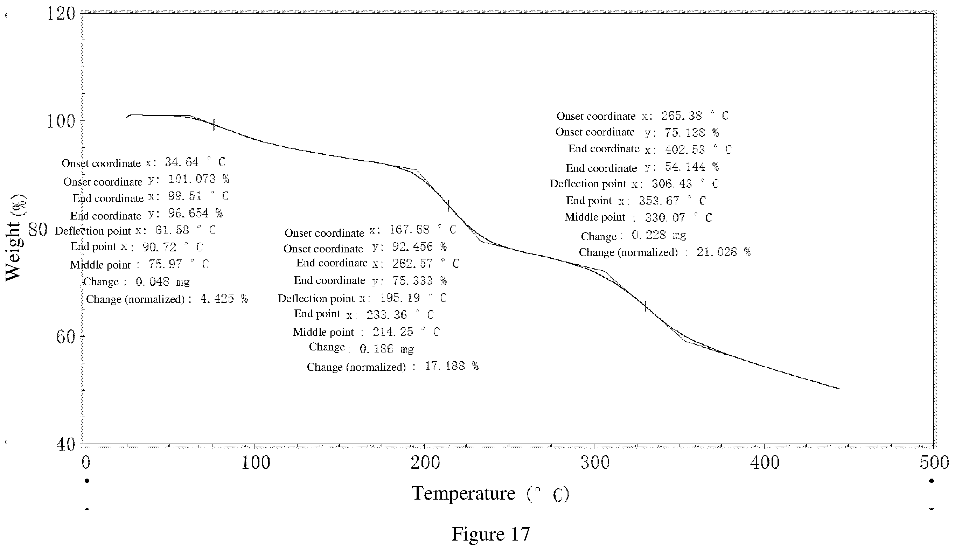

15 mg of the compound of formula I was dissolved in 1 ml of DMF, and L-tartaric acid (6.4mg) was added thereto. The solution was heated to 50°C and stirred for 4 hours at the constant temperature of 50°C, then cooled to 0°C and stirred for two days or longer at the controlled temperature to continue the reaction. After drying under vacuum, the solid obtained was determined as crystal form E of L-tartrate salt of the compound of formula I by XRPD. The XRPD detection data of crystal form E of L-tartrate salt of the compound of formula I are shown in Table 7, and its XRPD pattern is shown in Figure 15. Its DSC spectrum is shown in Figure 16 with an endothermic peak at 132°C. Its TGA spectrum is shown in Figure 17.

**Table 7. Data of XRPD characteristic diffraction peaks of crystal form E of L-tartrate salt of the compound of formula I**

| Peak No. | 2θ[° or degree] | d[Å] | Relative strength |
|---|---|---|---|
| 1 | 5.280 | 16.73882 | 5.35 |
| 2 | 6.383 | 13.84805 | 100 |
| 3 | 7.659 | 11.54289 | 2.26 |
| 4 | 9.081 | 9.73825 | 17.53 |
| 5 | 10.562 | 8.37572 | 4.6 |
| 6 | 12.936 | 6.84352 | 28.29 |
| 7 | 13.677 | 6.47442 | 8.89 |
| 8 | 14.400 | 6.15097 | 18.35 |
| 9 | 15.501 | 5.71646 | 5.38 |
| 10 | 16.161 | 5.48458 | 56.84 |
| 11 | 17.429 | 5.08821 | 8.92 |
| 12 | 18.397 | 4.82273 | 60.97 |
| 13 | 19.489 | 4.55499 | 11.09 |
| 14 | 20.790 | 4.27278 | 5.14 |
| 15 | 21.521 | 4.12913 | 6.23 |
| 16 | 22.563 | 3.94072 | 8.1 |
| 17 | 23.786 | 3.74092 | 29.6 |
| 18 | 24.536 | 3.62813 | 25.97 |
| 19 | 25.303 | 3.51995 | 4.26 |
| 20 | 26.203 | 3.40107 | 42 |
| 21 | 28.813 | 3.09859 | 6.38 |
| 22 | 29.430 | 3.03503 | 2.68 |
| 23 | 30.664 | 2.91571 | 4.47 |
| 24 | 31.693 | 2.82335 | 3.6 |
| 25 | 33.223 | 2.69668 | 5.1 |

### Example 8: Preparation of crystal form F of hydrobromide salt of the compound of formula I

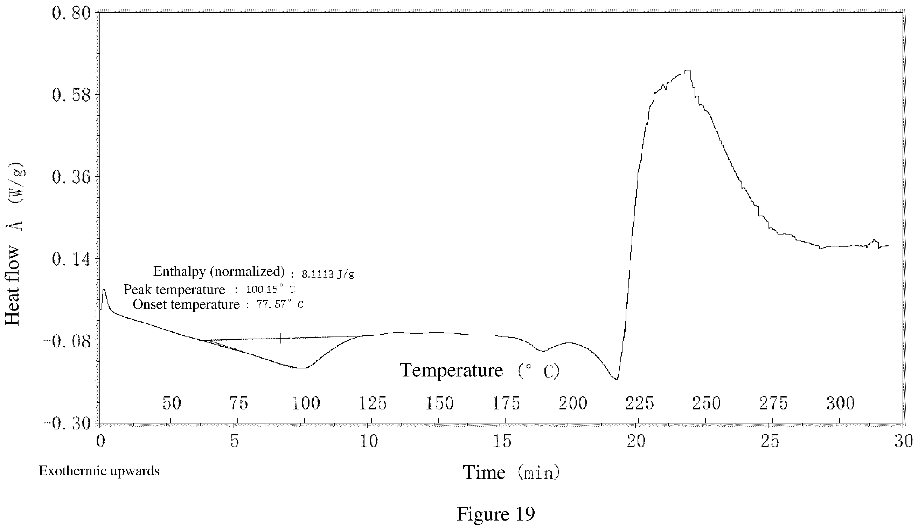

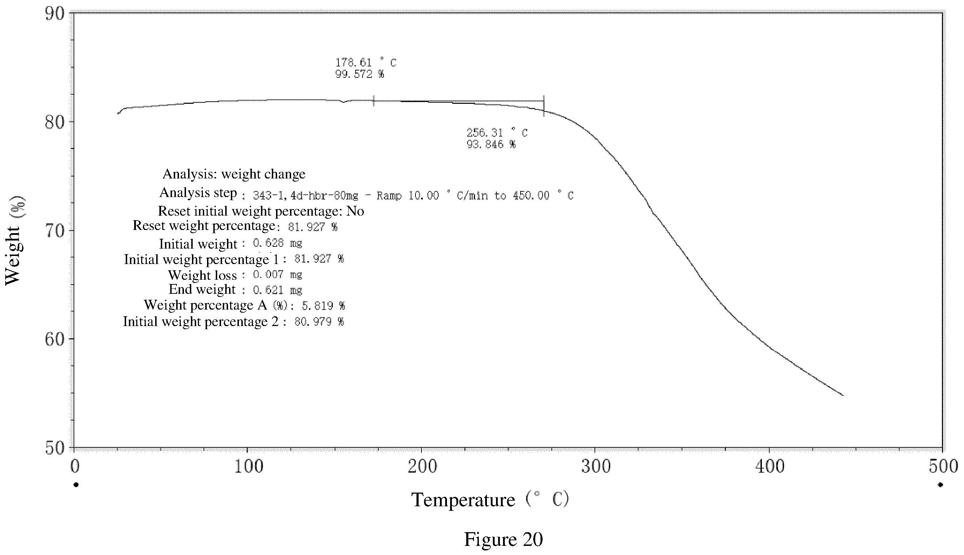

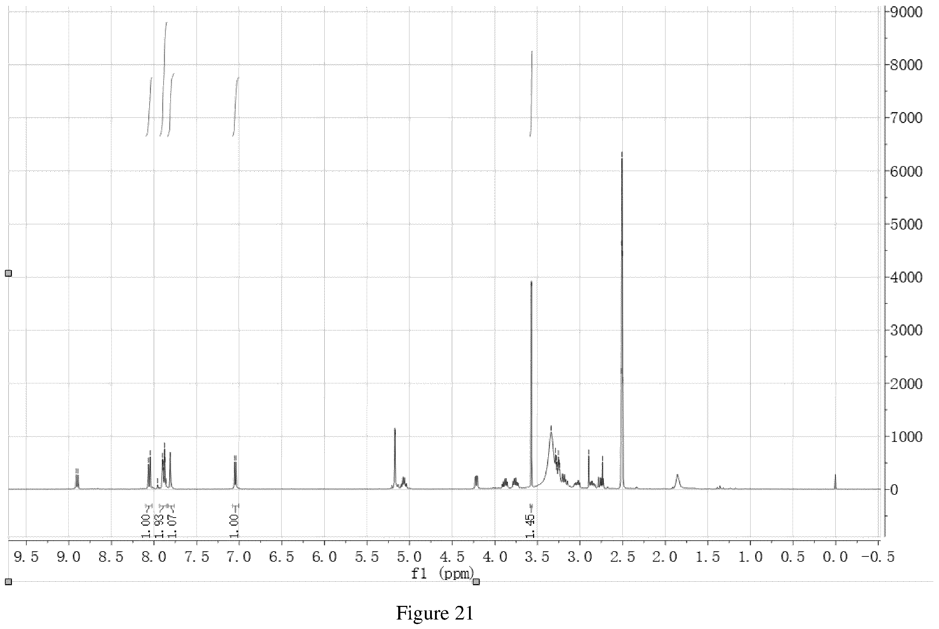

80 mg of the compound of formula I was dissolved in 1 ml of dioxane, and hydrobromic acid was added thereto in a molar ratio of 1.2:1 of the acid to API. A solid was precipitated under stirring for 12 hours at room temperature. After drying under vacuum, the solid obtained was determined as crystal form F of hydrobromide salt of the compound of formula I by XRPD. The XRPD detection data are shown in Table 8, and its XRPD pattern is shown in Figure 18. Its DSC spectrum is shown in Figure 19 with endothermic peaks at 99°C and 216°C. Its TGA spectrum is shown in Figure 20. The NMR result (Figure 21) showed a salt-forming with the compound I and hydrobromic acid in a ratio of approximately 1: 1.

**Table 8. Data of XRPD characteristic diffraction peaks of crystal form F of hydrobromide salt of the compound of formula I**

| Peak No. | 2θ[° or degree] | d[Å] | Relative strength |
|---|---|---|---|
| 1 | 6.152 | 14.36608 | 8.33 |
| 2 | 7.133 | 12.39334 | 13.83 |
| 3 | 10.827 | 8.17165 | 8.44 |
| 4 | 12.485 | 7.08993 | 38.96 |
| 5 | 14.422 | 6.14156 | 100 |
| 6 | 17.721 | 5.0051 | 31.01 |
| 7 | 18.823 | 4.71444 | 11.13 |
| 8 | 19.475 | 4.55823 | 2.36 |
| 9 | 20.738 | 4.28337 | 12.54 |
| 10 | 21.656 | 4.10375 | 26.42 |
| 11 | 22.309 | 3.98505 | 19.99 |
| 12 | 23.238 | 3.82781 | 23.67 |
| 13 | 25.116 | 3.54578 | 25.38 |
| 14 | 25.672 | 3.47023 | 24.4 |
| 15 | 27.416 | 3.25331 | 25.67 |
| 16 | 29.566 | 3.02144 | 16.76 |
| 17 | 33.028 | 2.71217 | 10.17 |
| 18 | 34.566 | 2.59498 | 4.43 |
| 19 | 36.479 | 2.46313 | 17 |
| 20 | 43.194 | 2.09449 | 10.58 |
| 21 | 44.683 | 2.02812 | 10.15 |

### Example 9: Stability test of crystal form A of hydrochloride salt of the compound of formula I

It is determined whether light, temperature and humidity have an influence on the stability of crystal form A of hydrochloride salt of the compound of formula I. The crystal form A of hydrochloride salt was placed in a stabilizing chamber in the dark and subjected to influence factor tests using a series of temperature and humidity combinations. After being placed for different days under light and various temperature and humidity conditions, the samples were taken out and compared with the initial crystal form A of hydrochloride salt. According to the determination by high performance liquid chromatography, none of them had a significant increase in isomers of the compound of formula I, as shown in Table 9. The samples taken out on the last day under light and various temperature and humidity conditions were subj ected to XRPD detection, showing that the crystal form A of hydrochloride salt had no change and had good stability.

**Table 9. Determination results of isomers in influence factor tests of crystal form A of hydrochloride salt**

| Name | Content % | Isomers % |
|---|---|---|
| Crystal form A of hydrochloride salt - initial | 99.54 | 0.67 |
| Crystal form A of hydrochloride salt - 0.5 ICH | 99.44 | 0.67 |
| Crystal form A of hydrochloride salt - 1 ICH | 99.32 | 0.67 |
| Crystal form A of hydrochloride salt - 25°C 92% - one week | 99.37 | 0.63 |
| Crystal form A of hydrochloride salt - 25°C 92% - two weeks | 99.31 | 0.70 |
| Crystal form A of hydrochloride salt - 60°C - one week | 99.31 | 0.70 |
| Crystal form A of hydrochloride salt - 60°C - two weeks | 99.33 | 0.67 |
| Crystal form A of hydrochloride salt - 40°C 75% - one week | 99.31 | 0.69 |
| Crystal form A of hydrochloride salt - 40°C 75% - two weeks | 99.32 | 0.68 |

### Example 10: Preparation of crystal form A' of hydrochloride salt of the compound of formula I

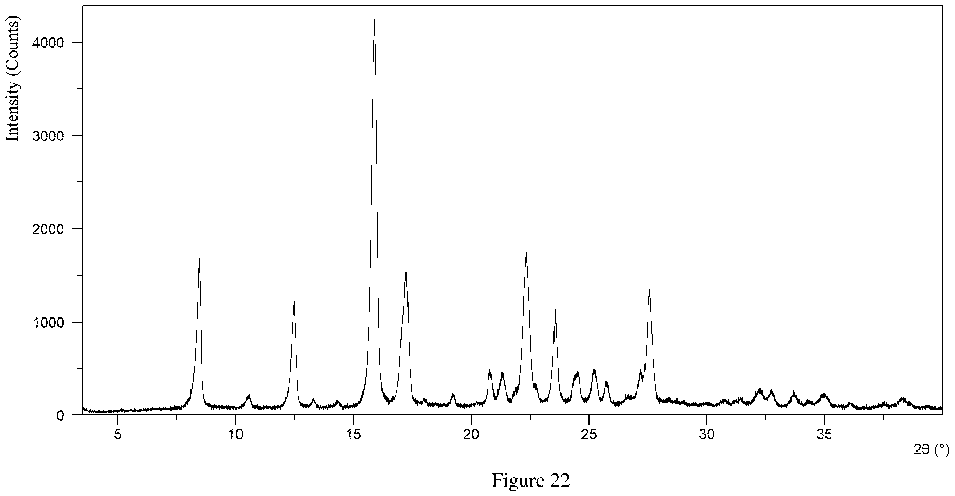

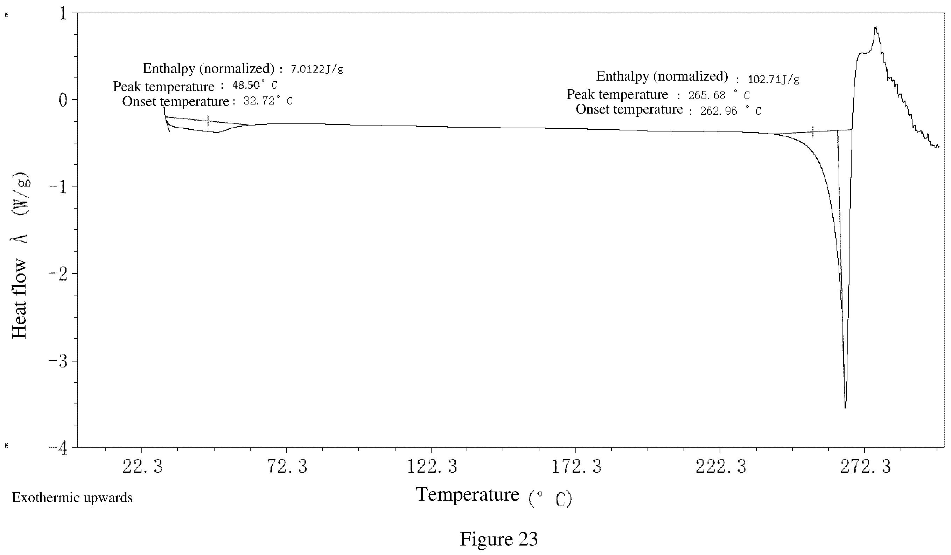

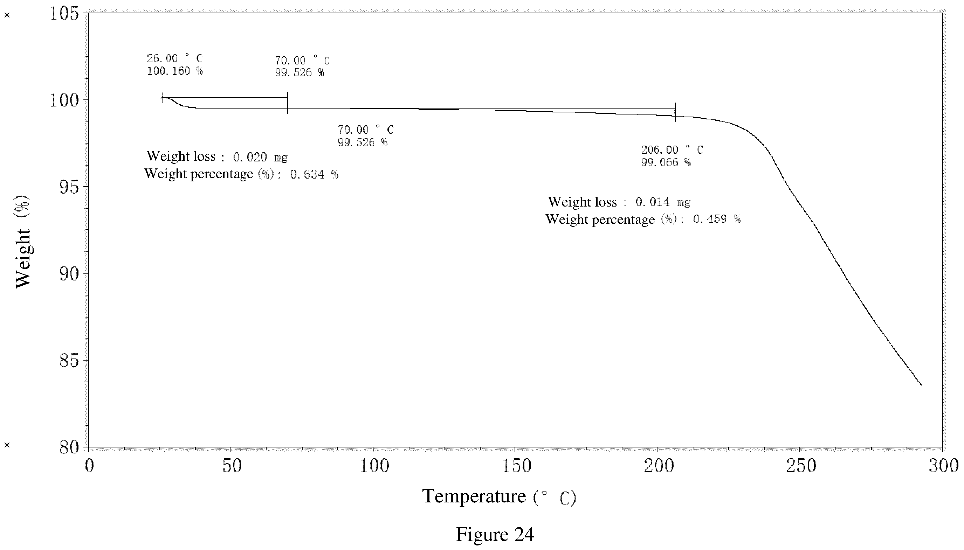

0.42 g (0.1 mmol) of the compound of formula I was weighed and added to 25 ml of ethanol, stirred at 70°C, and after the solution was clear, filtered. A white solid was precipitated from the filtrate. The filtrate-containing bottle was transferred to an oil bath at 70°C, and after the solution was clear, 2 ml of a solution of hydrochloric acid and ethanol (1: 1) was added thereto to precipitate a large amount of solid. The mixture was cooled to room temperature, subjected to crystallization for 4 hours, and filtered. The filter cake was dried for 12 hours at 50°C to obtain 0.39 g of crystal form A' with a yield of 85.5%. Its XRPD pattern is shown in Figure 22, and the positions of the characteristic peaks thereof are shown in Table 10. Its DSC spectrum is shown in Figure 23 with an endothermic peak at 161°C, showing low crystallinity. Its TGA spectrum is shown in Figure 24.

**Table 10. Data of XRPD characteristic diffraction peaks of crystal form A' of hydrochloride salt of the compound of formula I**

| Peak No. | 2*θ*[° or degree] | d[Å] | Relative strength |
|---|---|---|---|
| 1 | 8.452 | 10.461 | 38.21 |
| 2 | 10.537 | 8.396 | 2.98 |
| 3 | 12.476 | 7.095 | 27.18 |
| 4 | 13.296 | 6.659 | 1.79 |
| 5 | 14.332 | 6.180 | 1.27 |
| 6 | 15.884 | 5.580 | 100.00 |
| 7 | 17.037 | 5.204 | 20.37 |
| 8 | 17.227 | 5.148 | 34.21 |
| 9 | 17.997 | 4.929 | 1.51 |
| 10 | 19.227 | 4.616 | 2.55 |
| 11 | 20.784 | 4.274 | 8.83 |
| 12 | 21.329 | 4.166 | 7.73 |
| 13 | 22.328 | 3.982 | 38.83 |
| 14 | 22.775 | 3.905 | 4.39 |
| 15 | 23.566 | 3.775 | 24.02 |
| 16 | 24.353 | 3.655 | 6.41 |
| 17 | 24.497 | 3.634 | 7.98 |
| 18 | 25.221 | 3.531 | 8.88 |
| 19 | 25.742 | 3.461 | 5.71 |
| 20 | 26.663 | 3.343 | 1.96 |
| 23 | 27.175 | 3.282 | 8.42 |
| 24 | 27.567 | 3.236 | 29.26 |
| 25 | 28.354 | 3.148 | 1.42 |
| 26 | 30.728 | 2.91 | 1.44 |
| 27 | 31.430 | 2.846 | 1.71 |
| 28 | 32.228 | 2.778 | 3.87 |
| 29 | 32.742 | 2.735 | 3.74 |
| 30 | 33.688 | 2.661 | 3.29 |
| 31 | 34.352 | 2.611 | 1.43 |
| 32 | 34.960 | 2.567 | 3.20 |
| 33 | 36.079 | 2.490 | 1.07 |
| 34 | 37.518 | 2.397 | 0.90 |
| 35 | 38.300 | 2.350 | 2.41 |

## Claims

1. A pharmaceutically acceptable salt of a compound of formula I, wherein the pharmaceutically acceptable salt is an acid addition salt selected from the group consisting of hydrochloride salt, methanesulfonate salt, phosphate salt, L-tartrate salt, maleate salt, p-toluenesulfonate salt, sulfate salt, fumarate salt, succinate salt, citrate salt, malate salt and hydrobromide salt, and is preferably in the form of a crystal,

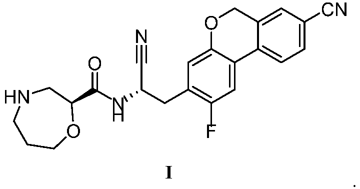

2. The pharmaceutically acceptable salt of a compound of formula I according to claim 1, wherein the stoichiometric ratio of the compound of formula I to the acid molecule or acid group is from 1:0.5 to 1:3, preferably 1:0.5, 1:1, 1:1.2, 1:2 or 1:3;
specially,
the pharmaceutically acceptable salt of the compound of formula I is hydrochloride salt, and the stoichiometric ratio of the compound of formula I to the hydrochloric acid group is 1:1.

3. A method for preparing the pharmaceutically acceptable salt of a compound of formula I according to claim 1 or 2, comprising a step of salifying the compound of formula I with an acid;
specially,
the step of salifying the compound of formula I with an acid is carried out in a solvent selected from one or more of dichloromethane, N,N-dimethylformamide, acetonitrile, methanol, ethanol, isopropanol, tetrahydrofuran, acetone, 1,4-dioxane, water, dimethyl sulfoxide, and ethyl acetate, preferably acetone, methanol, ethanol, ethanol/water, isopropanol/water, ethyl acetate, acetonitrile, tetrahydrofuran, dimethyl sulfoxide/water, 1,4-dioxane, methanol/water, N,N-dimethylformamide, and dichloromethane.

4. Crystal form A of hydrochloride salt of a compound of formula I, wherein the X-ray powder diffraction pattern thereof represented by diffraction angle 2*θ* angle has characteristic peaks at 7.043, 12.430, 14.356, 14.840 and 15.250, preferably has characteristic peaks at 7.043, 8.719, 10.737, 12.430, 14.356, 14.840, 15.250 and 17.686, more preferably has characteristic peaks at 7.043, 8.719, 10.737, 12.430, 14.356, 14.840, 15.250, 17.686, 21.697, 22.305, 25.635 and 27.433, wherein the error range of the 2*θ* angle is ±0.20,

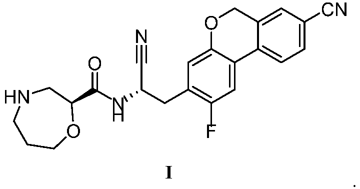

5. The crystal form A of hydrochloride salt of the compound of formula I according to claim 4, wherein the differential scanning calorimetry (DSC) spectrum thereof has an endothermic peak at 268°C, with the error range of ±2°C.

6. Crystal form A' of hydrochloride salt of a compound of formula I, wherein the X-ray powder diffraction pattern thereof represented by diffraction angle 2*θ* angle has characteristic peaks at 8.452, 12.476, 15.884, 17.037, 17.227, 22.328 and 23.566, preferably has characteristic peaks at 8.452, 10.537, 12.476, 15.884, 17.037, 17.227, 20.784, 22.328, 23.566 and 27.567, more preferably has characteristic peaks at 8.452, 10.537, 12.476, 13.296, 14.332, 15.884, 17.037, 17.227, 20.784, 22.328, 23.566 and 27.567, most preferably has characteristic peaks at 8.452, 10.537, 12.476, 13.296, 14.332, 15.884, 17.037, 17.227, 20.784, 21.329, 22.328, 23.566, 24.497, 25.221 and 27.567, wherein the error range of the 2*θ* angle is ±0.20,

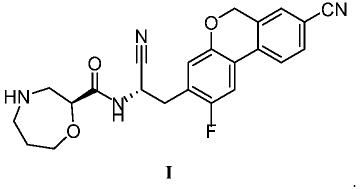

7. The crystal form A' of hydrochloride salt of the compound of formula I according to claim 6, wherein the DSC spectrum thereof has an endothermic peak at 263°C, with the error range of ±2°C.

8. Crystal form B of methanesulfonate salt of a compound of formula I, wherein the X-ray powder diffraction pattern thereof represented by diffraction angle 2*θ* angle has characteristic peaks at 6.717, 8.783, 13.969, 15.902, 16.647 and 17.515, preferably has characteristic peaks at 6.717, 8.105, 8.783, 13.969, 15.902, 16.647 and 17.515, more preferably has characteristic peaks at 6.717, 8.105, 8.783, 13.969, 15.902, 16.647, 17.515, 20.446, 21.069, 21.645 and 24.665, most preferably has characteristic peaks at 6.717, 8.105, 8.783, 13.969, 15.902, 16.647, 17.515, 20.446, 21.069, 21.645, 23.549, 24.665, 26.284, 27.289 and 27.667, wherein the error range of the 2*θ* angle is ±0.20,

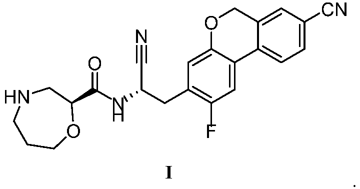

9. The crystal form B of methanesulfonate salt of the compound of formula I according to claim 8, wherein the DSC spectrum thereof has an endothermic peak at 161°C, with the error range of ±2°C.

10. Crystal form C of methanesulfonate salt of a compound of formula I, wherein the X-ray powder diffraction pattern thereof represented by diffraction angle 2*θ* angle has characteristic peaks at 7.747, 11.163, 12.676, 15.268, 16.824, 18.549 and 19.759, preferably has characteristic peaks at 4.522, 7.747, 11.163, 12.676, 15.268, 16.824, 18.549 and 19.759, more preferably has characteristic peaks at 4.522, 7.747, 11.163, 12.676, 15.268, 16.824, 18.549, 19.759, 21.460, 24.637 and 25.497, most preferably has characteristic peaks at 4.522, 7.747, 11.163, 12.676, 15.268, 16.824, 18.549, 19.759, 21.460, 22.539, 24.637 and 25.497, wherein the error range of the 2*θ* angle is ±0.20,

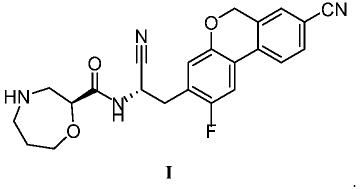

11. The crystal form C of methanesulfonate salt of the compound of formula I according to claim 10, wherein the DSC spectrum thereof has an endothermic peak at 194°C, with the error range of ±2°C.

12. Crystal form D of phosphate salt of a compound of formula I, wherein the X-ray powder diffraction pattern thereof represented by diffraction angle 2*θ* angle has characteristic peaks at 9.593, 12.831, 13.464, 15.666, 18.161 and 19.245, preferably has characteristic peaks at 8.491, 9.593, 12.831, 13.464, 13.954, 14.943, 15.666, 16.616, 17.259, 18.161 and 19.245, more preferably has characteristic peaks at 4.213, 6.650, 8.491, 9.593, 10.897, 12.831, 13.464, 13.954, 14.943, 15.666, 16.616, 17.259, 18.161 and 19.245, most preferably has characteristic peaks at 4.213, 6.650, 8.491, 9.593, 10.897, 12.831, 13.464, 13.954, 14.943, 15.666, 16.616, 17.259, 18.161, 19.245, 24.822, 25.665 and 26.618, wherein the error range of the 2*θ* angle is ±0.20,

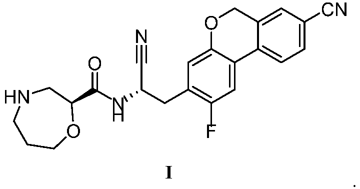

13. The crystal form D of phosphate salt of the compound of formula I according to claim 12, wherein the DSC spectrum thereof has endothermic peaks at 130°C and 143°C, with the error range of ±2°C.

14. Crystal form E of L-tartrate salt of a compound of formula I, wherein the X-ray powder diffraction pattern thereof represented by diffraction angle 2*θ* angle has characteristic peaks at 6.383, 9.081, 12.936, 16.161 and 18.397, preferably has characteristic peaks at 6.383, 9.081, 12.936, 14.400, 16.161, 18.397 and 19.489, more preferably has characteristic peaks at 6.383, 9.081, 12.936, 14.400, 16.161, 18.397, 19.489, 23.786, 24.536 and 26.203, wherein the error range of the 2*θ* angle is ±0.20,

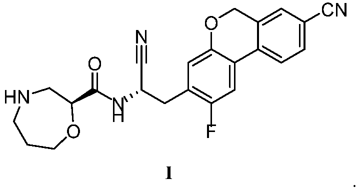

15. The crystal form E of L-tartrate salt of the compound of formula I according to claim 14, wherein the DSC spectrum thereof has an endothermic peak at 132°C, with the error range of ±2°C.

16. Crystal form F of hydrobromide salt of a compound of formula I, wherein the X-ray powder diffraction pattern thereof represented by diffraction angle 2*θ* angle has characteristic peaks at 7.133, 12.485, 14.422, 17.721 and 18.823, preferably has characteristic peaks at 6.152, 7.133, 10.827, 12.485, 14.422, 17.721 and 18.823, more preferably has characteristic peaks at 6.152, 7.133, 10.827, 12.485, 14.422, 17.721, 18.823, 20.738, 21.656, 22.309, 23.238, 25.116, 25.672 and 27.416, wherein the error range of the 2*θ* angle is ±0.20,

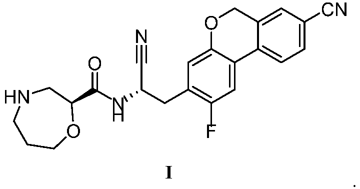

17. The crystal form F of hydrobromide salt of the compound of formula I according to claim 16, wherein the DSC spectrum thereof has endothermic peaks at 99°C and 216°C, with the error range of ±2°C.

18. A pharmaceutical composition comprising the pharmaceutically acceptable salt of the compound of formula I according to claim 1 or 2 or the crystal form according to any one of claims 4-17, and a pharmaceutically acceptable excipient.

19. Use of the pharmaceutically acceptable salt of a compound of formula I according to claim 1 or 2, the crystal form according to any one of claims 4-17 or the pharmaceutical composition according to claim 18, in the preparation of a medicament for preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa,, or cancer.
